# EUROPEAN PATENT APPLICATION

(11) **EP 4 361 143 A1**
(43) Date of publication of application: **01.05.2024**
(21) Application number: 22864456.3
(22) Date of filing: 29.08.2022
(51) Int. Cl.: C07D 401/14

(54) **METHOD FOR PRODUCING MONOCYCLIC PYRIDINE DERIVATIVE**

(30) Priority: 31.08.2021 JP 2021141386
(71) Applicant: Eisai R&D Management Co., Ltd., Tokyo 112-8088 (JP)
(72) Inventor: NAGAI, Mitsuo, Kamisu-shi, Ibaraki 314-0255 (JP); FUKUYAMA, Takashi, Tsukuba-shi, Ibaraki 300-2635 (JP); KAMADA, Yasuaki, Tsukuba-shi, Ibaraki 300-2635 (JP); NIIJIMA, Jun, Tsukuba-shi, Ibaraki 300-2635 (JP); KURODA, Hirofumi, Kamisu-shi, Ibaraki 314-0255 (JP); MURAKAMI, Keiichi, Kamisu-shi, Ibaraki 314-0255 (JP); KAROJI, Yuki, Kamisu-shi, Ibaraki 314-0255 (JP); SAITO, Hiroyuki, Kamisu-shi, Ibaraki 314-0255 (JP); OMORI, Masayuki, Kamisu-shi, Ibaraki 314-0255 (JP); MIYASHITA, Yusuke, Kamisu-shi, Ibaraki 314-0255 (JP); KAMADA, Atsushi, Tsukuba-shi, Ibaraki 300-2635 (JP); MATSUDA, Masaaki, Tsukuba-shi, Ibaraki 300-2635 (JP); KODERA, Takanori, Kamisu-shi, Ibaraki 314-0255 (JP)
(74) Representative: Hoffmann Eitle
(86) International application number: PCT/JP2022/032310
(87) International publication number: WO 2023/032873

(57) **Abstract**

Provided is compound (3d) or a salt thereof, wherein the content of compound (IM-7) is 0.48 mass% or lower.

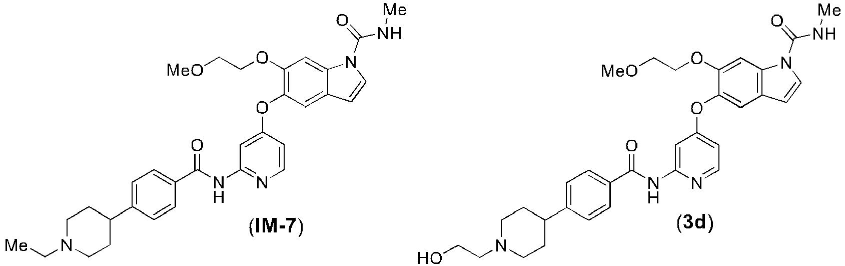

## Description

### Technical Field

The present invention relates to a process for producing monocyclic pyridine derivatives that are useful as FGFR inhibitors.

### Background Art

The monocyclic pyridine derivative 5-((2-(4-(1-(2-hydroxyethyl)piperidin-4-yl)benzamide)pyridin-4-yl)oxy)-6-(2-methoxyethox y)-N-methyl-1H-indole-1-carboxamide butanedioate (2:3) 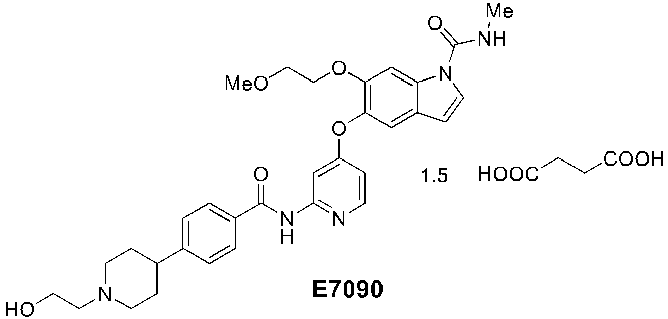 (hereunder also referred to as "E7090") has potent FGFR (Fibroblast Growth Factor Receptor) inhibitory action and is useful as a therapeutic agent for FGFR kinase-associated intrahepatic cholangiocarcinoma and breast cancer (PTLs 1 and 2).

### Citation List

### Patent Literature

[PTL 1] International Patent Publication No. WO2014/129477
[PTL 2] International Patent Publication No. WO2016/027781

### Summary of Invention

### Technical Problem

Processes for producing E7090 are described in PTL 1 (Example 22) and PTL 2 (Example 1).

For synthesis of compound (3d) described in PTL 1, improved modifications are desired because 1) preparation of the carboxylic acid chloride (compound (P 3-1)) using thionyl chloride is complicated, 2) by-products such as compound (IM-1) are observed during reaction between compound (2i) and the carboxylic acid chloride (compound (P 3-1)), and 3) trifluoroacetic acid is used as an acid catalyst in a methylene chloride solvent for conversion from compound (3b) to compound (3c), and both methylene chloride and trifluoroacetic acid are unsuitable for use on a commercial scale due to environmental considerations.

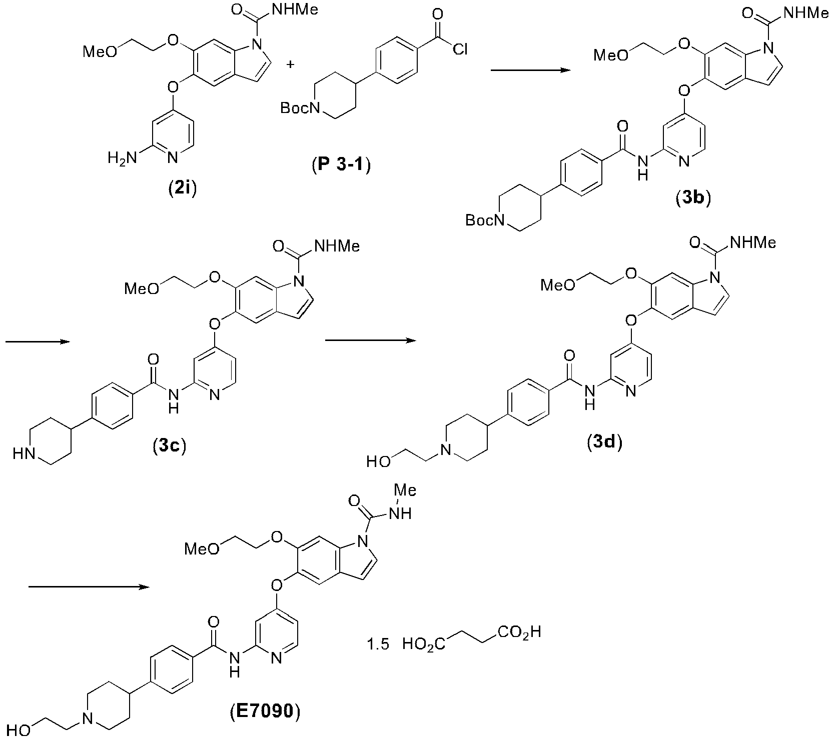

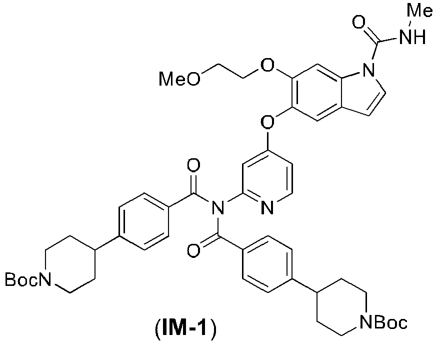

The present inventors have obtained the new knowledge that compound (2i), compound (3c), compound (IM-2), compound (IM-3), compound (IM-4), compound (IM-5), compound (IM-6), compound (IM-7) and compound (IM-8) are present as analogs in the production of E7090.

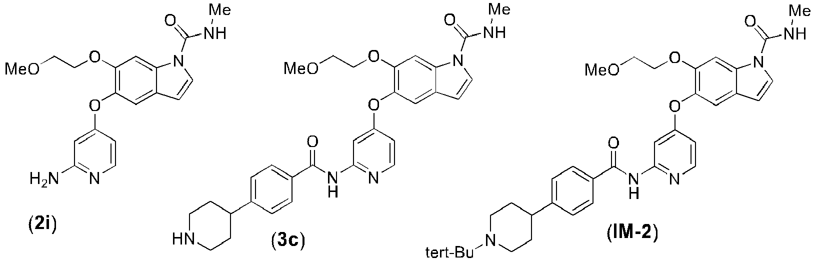

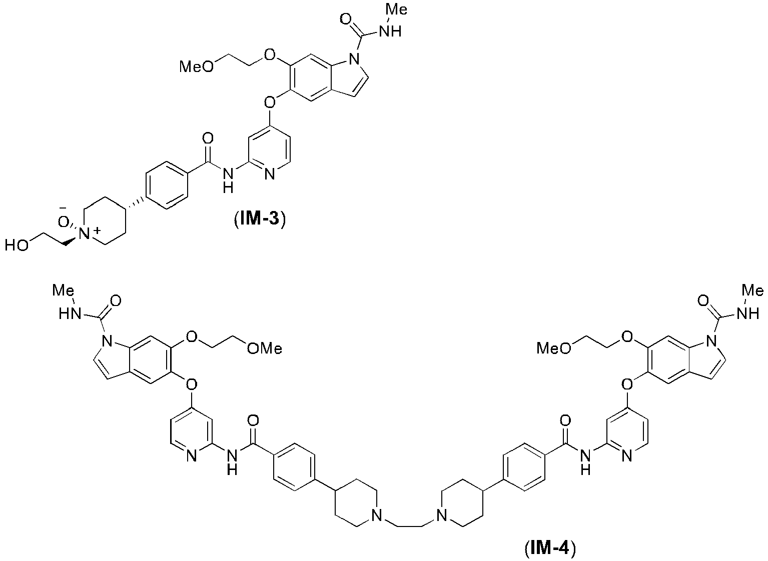

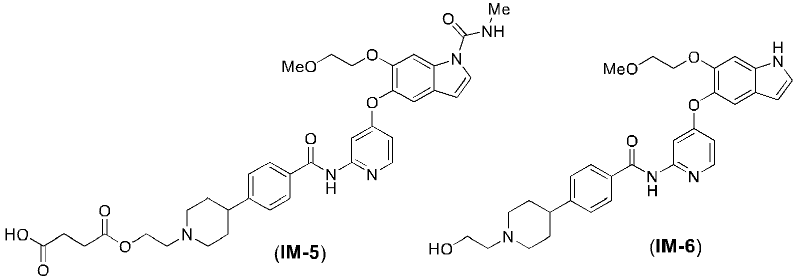

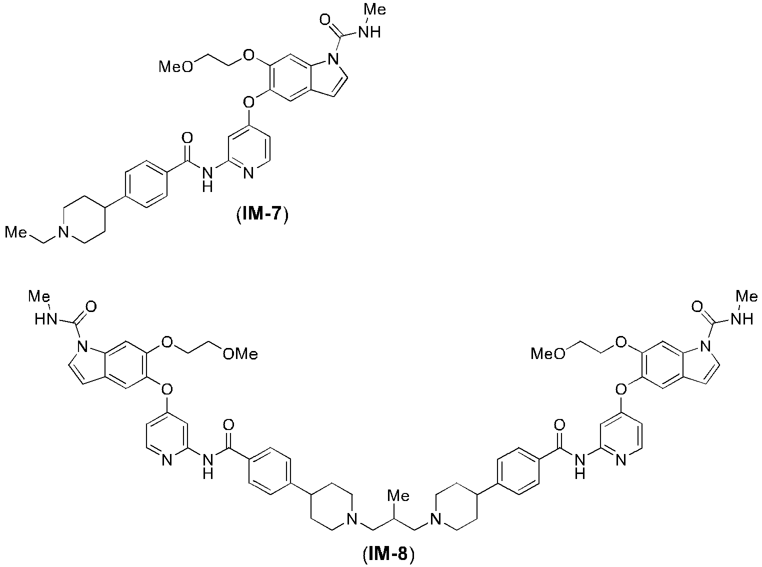

It is an object of the present application to provide a higher-yield, more efficient production process that allows synthesis of high-quality E7090 with low content of all analogs, as well as to provide the high-quality E7090.

### Solution to Problem

Provided herein is a production process useful for production of E7090, which is useful as an FGFR inhibitor. The present specification also provides E7090 with a low content of all analogs. Specifically, the present invention provides the following [1] to [54].
[1] A process for producing compound (3d): 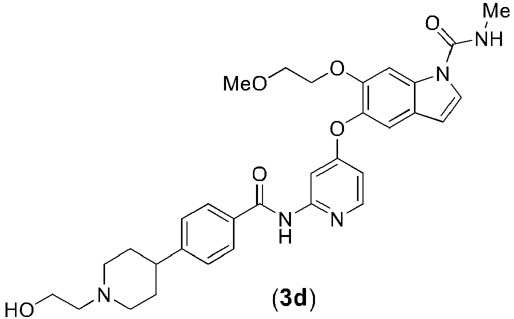 or a salt thereof, wherein the process comprises:
   a) step a) in which compound (2i): 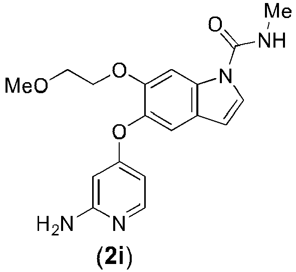 or a salt thereof and compound (3a): 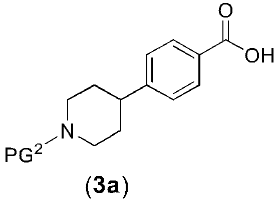 (where PG² represents a nitrogen protecting group)
      are reacted in the presence of a condensation agent, to produce compound (3b): 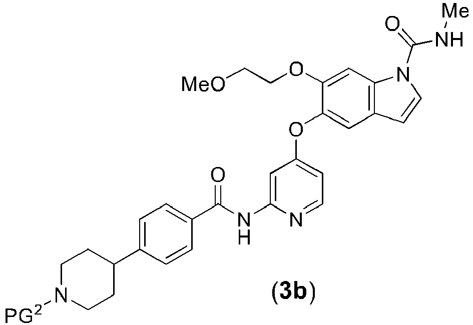 (where PG² represents the same group as specified above),
   b) step b) in which PG² in compound (3b) obtained in step a) is removed to produce compound (3c): 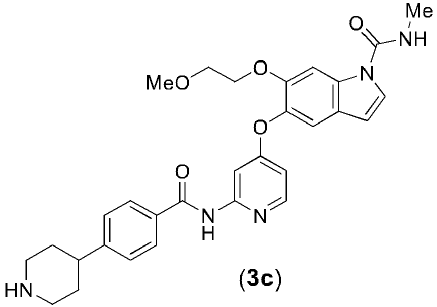
   c) step c) in which compound (3c) obtained in step b) and a hydroxyethylating agent are reacted to produce compound (3d): 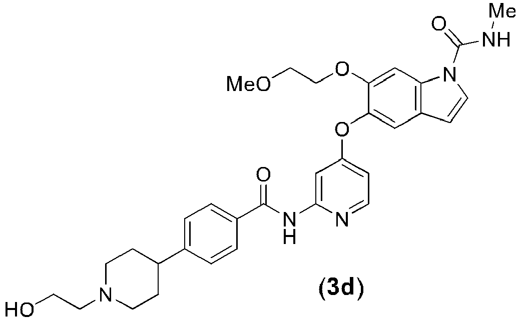 and
   d) if necessary, step d) in which compound (3d) obtained in step c) is converted to a pharmaceutically acceptable salt,
[2] The production process according to [1], wherein PG² is a tert-butoxycarbonyl group,
[3] The production process according to [1], wherein the condensation agent is 1-ethyl-3-(3-dimethylaminopropyl)carbodiimide hydrochloride,
[4] The production process according to [2], wherein formic acid or hydrochloric acid is used in step b),
[5] The production process according to [1], wherein the hydroxyethylating agent is 1,4-dioxane-2,5-diol, and step c) further includes the use of a reducing agent,
[6] The production process according to [1], wherein the pharmaceutically acceptable salt is a succinate,
[7] Compound (3d) or a salt thereof, wherein the content of compound (IM-7) is 0.48 mass% or lower, 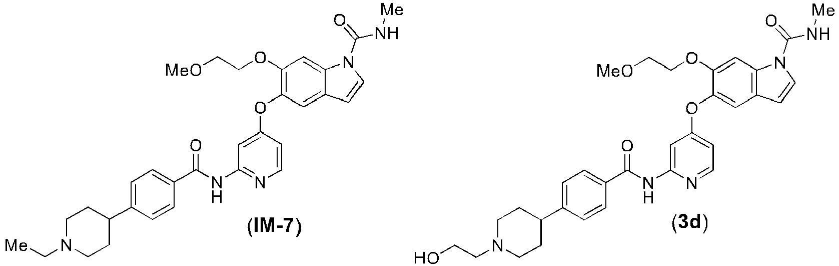
[8] Compound (3d) or a salt thereof, wherein the content of compound (IM-5) is 0.40 mass% or lower, 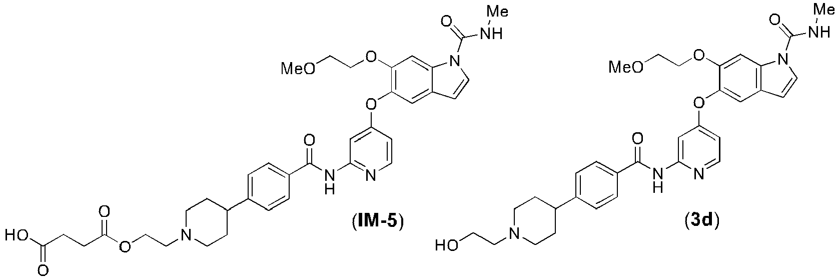
[9] Compound (3d) or a salt thereof, wherein the content of compound (IM-2) is 0.30 mass% or lower, 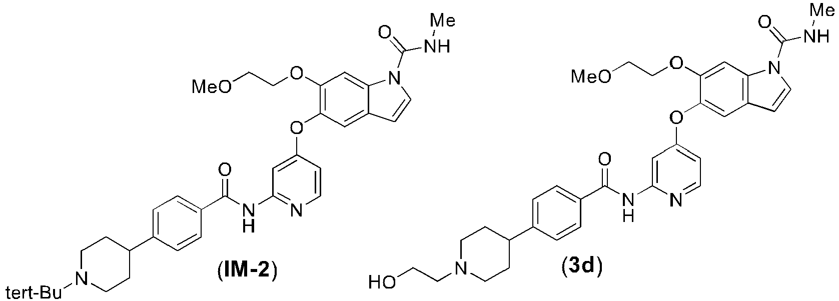
[10] Compound (3d) or a salt thereof, wherein the content of compound (IM-2) is 0.15 mass% or lower, 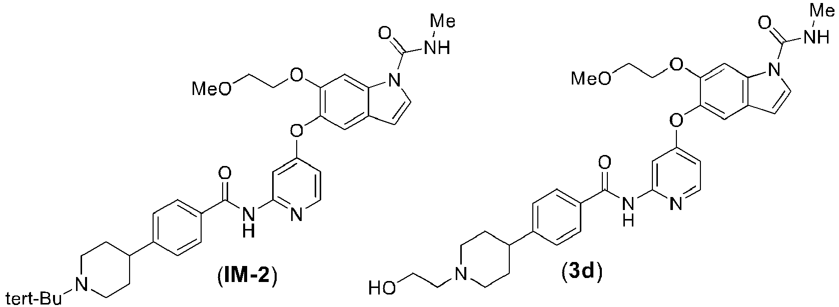
[11] Compound (3d) or a salt thereof, wherein the content of compound (3c) is 0.30 mass% or lower, 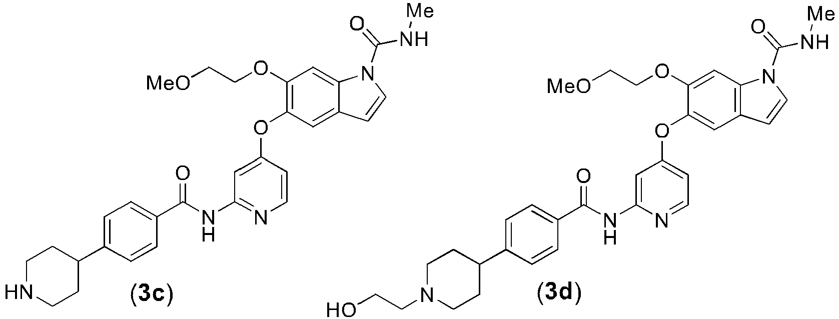
[12] Compound (3d) or a salt thereof, wherein the content of compound (IM-3) is 0.30 mass% or lower, 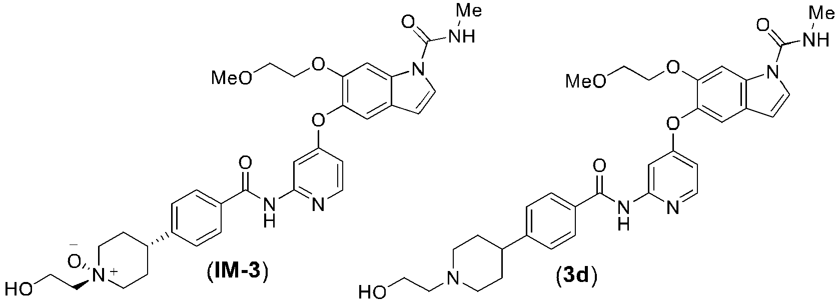
[13] Compound (3d) or a salt thereof, wherein the content of compound (IM-3) is 0.15 mass% or lower, 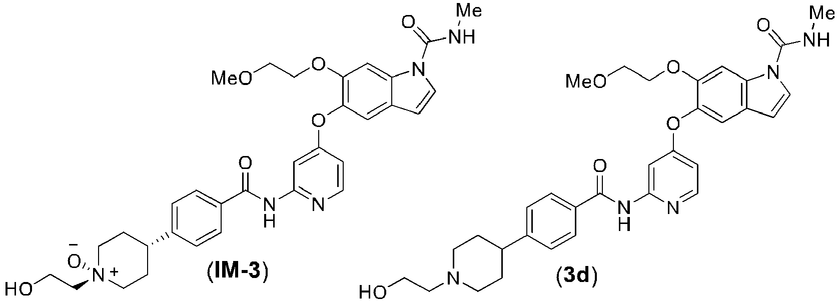
[14] Compound (3d) or a salt thereof, wherein the content of compound (2i) is 0.15 mass% or lower, 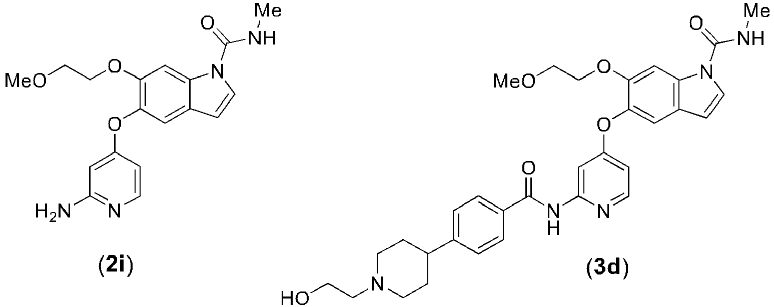
[15] Compound (3d) or a salt thereof, wherein the content of compound (IM-4) is 0.15 mass% or lower, 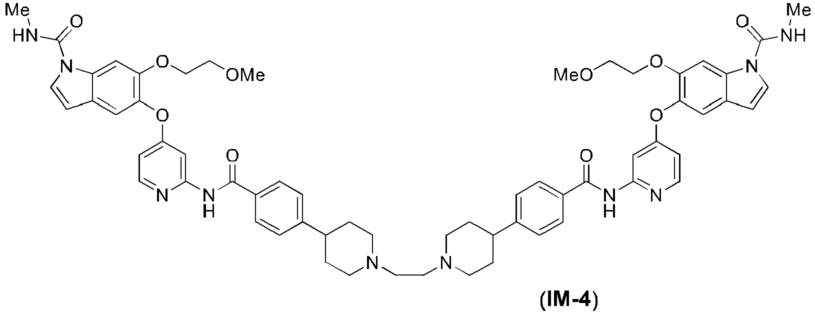 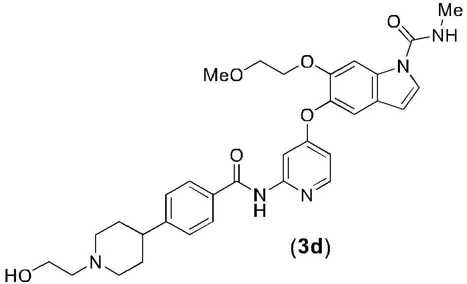
[16] Compound (3d) or a salt thereof, wherein the content of compound (IM-6) is 0.15 mass% or lower, 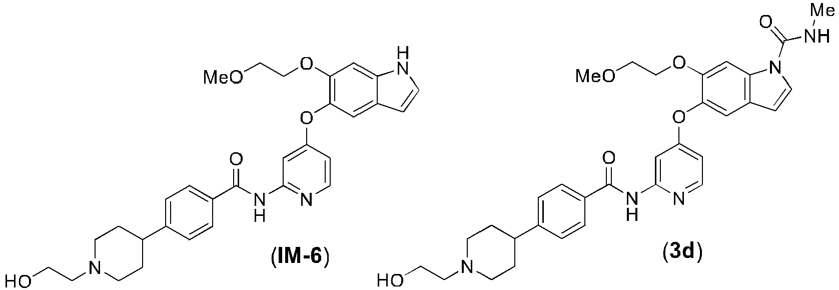
[17] Compound (3d) or a salt thereof, wherein the content of compound (IM-8) is 0.15 mass% or lower, 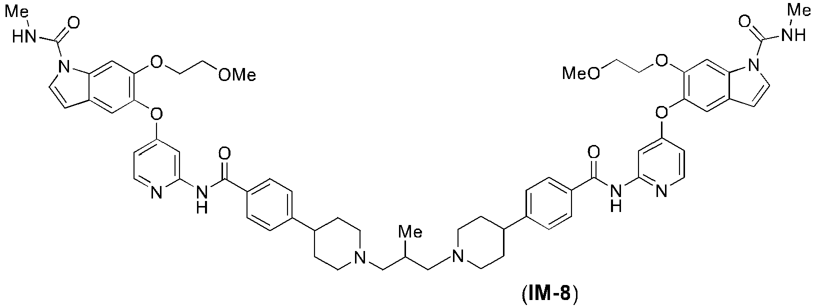 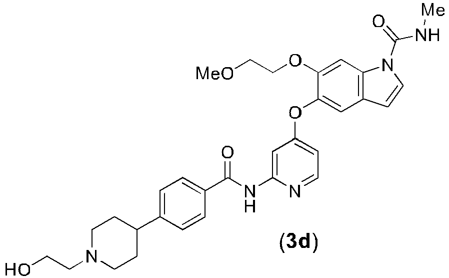
[18] Compound (3d) or a salt thereof, wherein the content of all analogs is 2.0 mass% or lower, 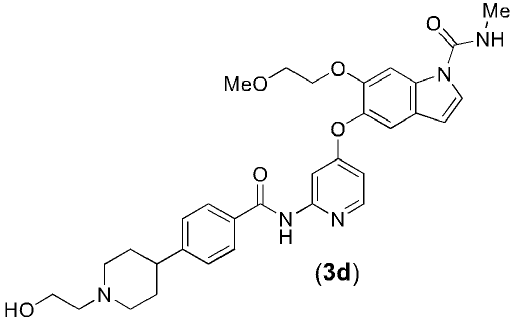
[19] Compound (3d) or a salt thereof, wherein the content of compound (IM-7) is 0.48 mass% or lower and the content of all analogs is 2.0 mass% or lower, 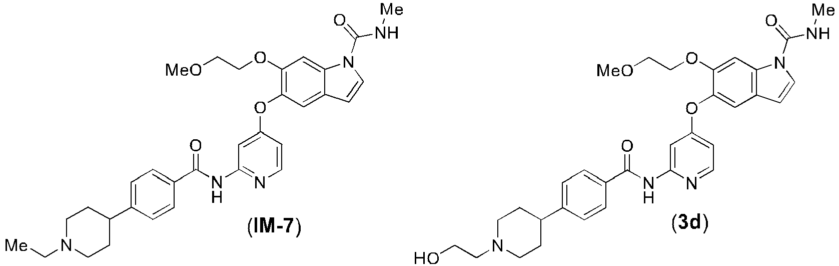
[20] Compound (3d) or a salt thereof, wherein the content of compound (IM-5) is 0.40 mass% or lower and the content of all analogs is 2.0 mass% or lower, 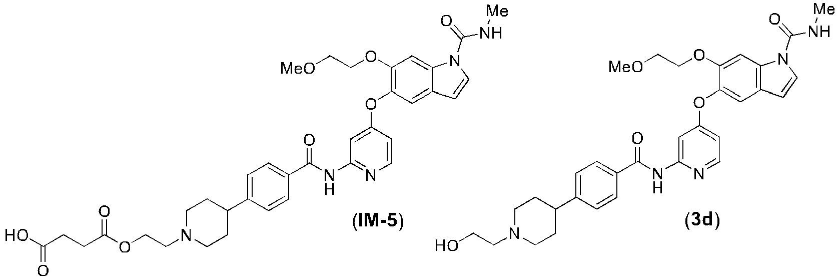
[21] Compound (3d) or a salt thereof, wherein the content of compound (IM-2) is 0.30 mass% or lower and the content of all analogs is 2.0 mass% or lower, 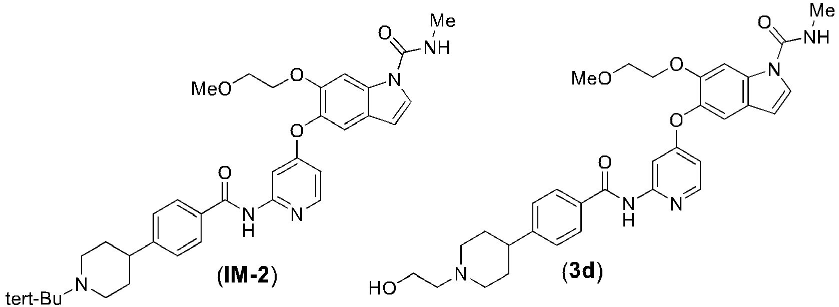
[22] Compound (3d) or a salt thereof, wherein the content of compound (IM-2) is 0.15 mass% or lower and the content of all analogs is 2.0 mass% or lower, 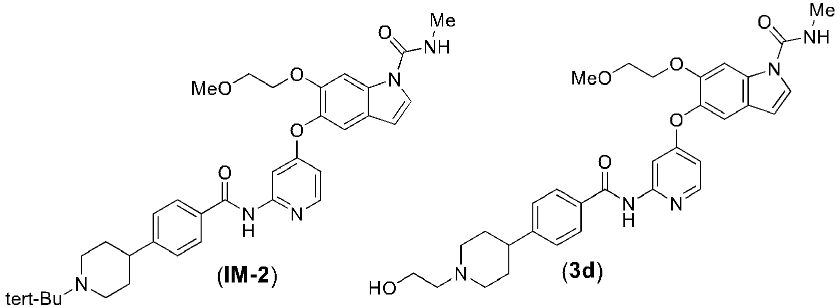
[23] Compound (3d) or a salt thereof, wherein the content of compound (3c) is 0.30 mass% or lower and the content of all analogs is 2.0 mass% or lower, 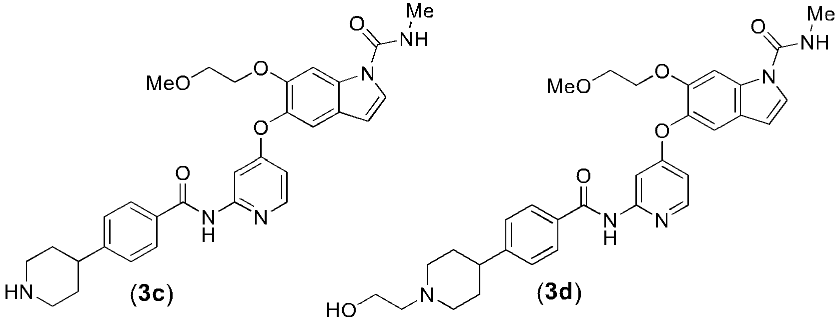
[24] Compound (3d) or a salt thereof, wherein the content of compound (IM-3) is 0.30 mass% or lower and the content of all analogs is 2.0 mass% or lower, 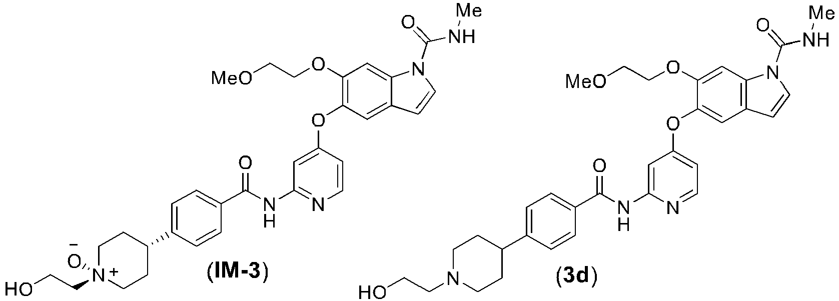
[25] Compound (3d) or a salt thereof, wherein the content of compound (IM-3) is 0.15 mass% or lower and the content of all analogs is 2.0 mass% or lower, 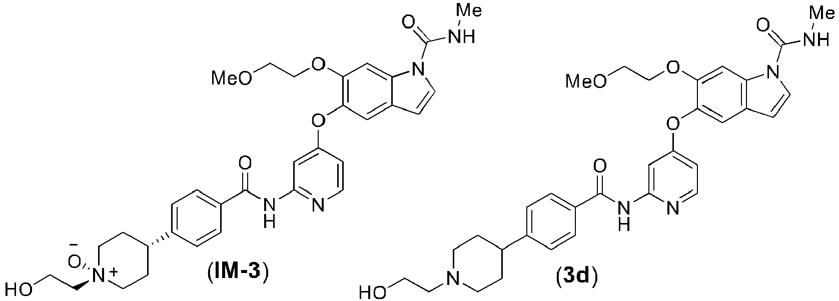
[26] Compound (3d) or a salt thereof, wherein the content of compound (2i) is 0.15 mass% or lower and the content of all analogs is 2.0 mass% or lower, 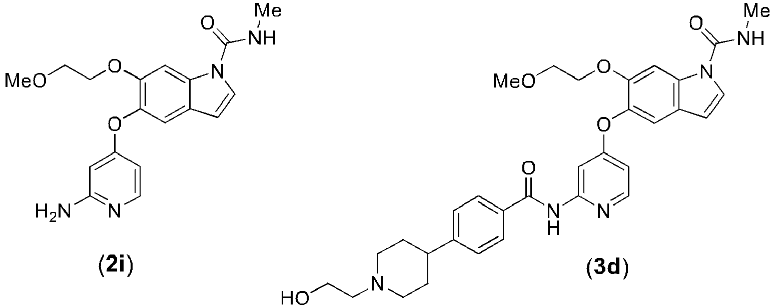
[27] Compound (3d) or a salt thereof, wherein the content of compound (IM-4) is 0.15 mass% or lower and the content of all analogs is 2.0 mass% or lower, 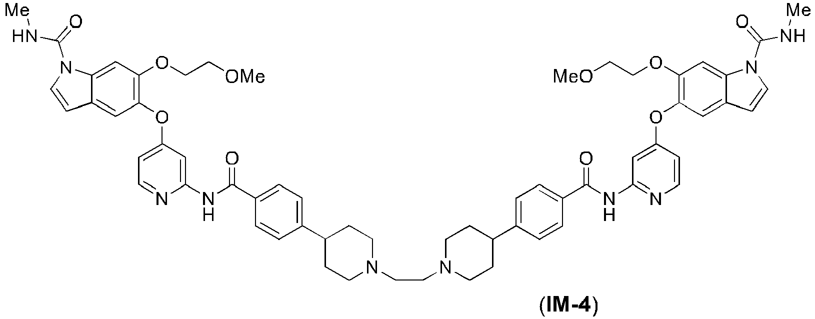 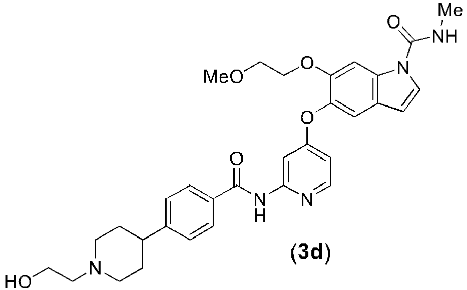
[28] Compound (3d) or a salt thereof, wherein the content of compound (IM-6) is 0.15 mass% or lower and the content of all analogs is 2.0 mass% or lower, 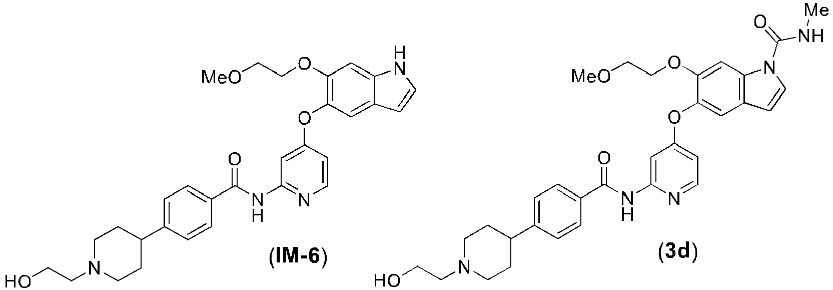
[29] Compound (3d) or a salt thereof, wherein the content of compound (IM-8) is 0.15 mass% or lower and the content of all analogs is 2.0 mass% or lower, 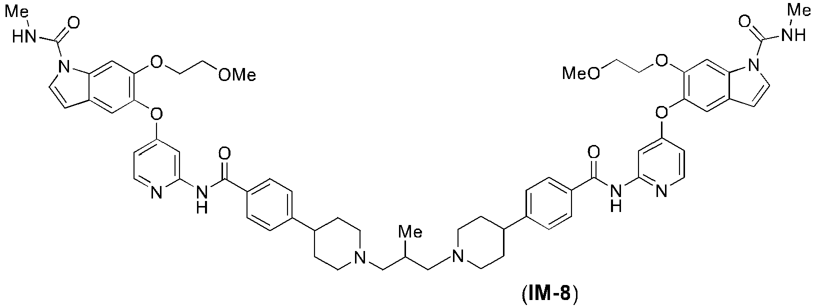 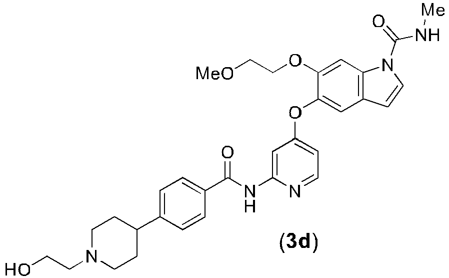
[30] Compound (3d) or a salt thereof, wherein the content of compound (3d) or a salt thereof is 97.0 mass% or greater, 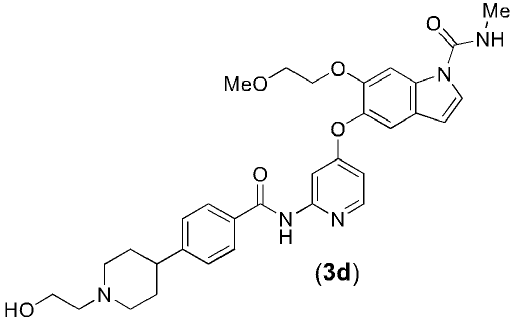
[31] Compound (3d) or a salt thereof, wherein the content of compound (3d) or a salt thereof is 97.0 mass% or greater and the content of compound (IM-7) is 0.48 mass% or lower, 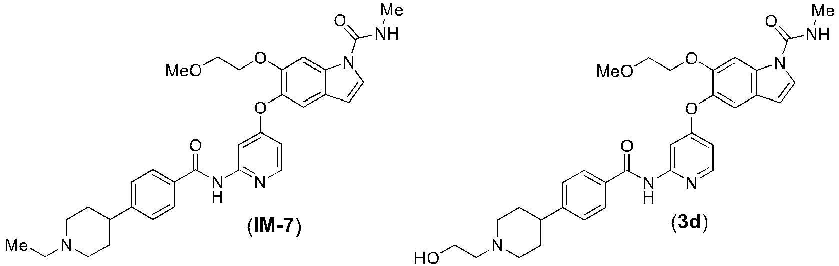
[32] Compound (3d) or a salt thereof, wherein the content of compound (3d) or a salt thereof is 97.0 mass% or greater and the content of compound (IM-5) is 0.40 mass% or lower, 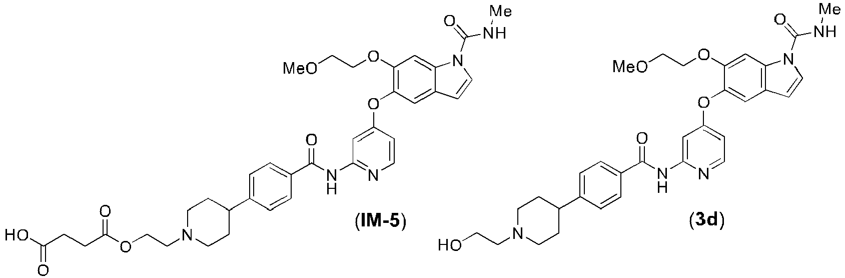
[33] Compound (3d) or a salt thereof, wherein the content of compound (3d) or a salt thereof is 97.0 mass% or greater and the content of compound (IM-2) is 0.30 mass% or lower, 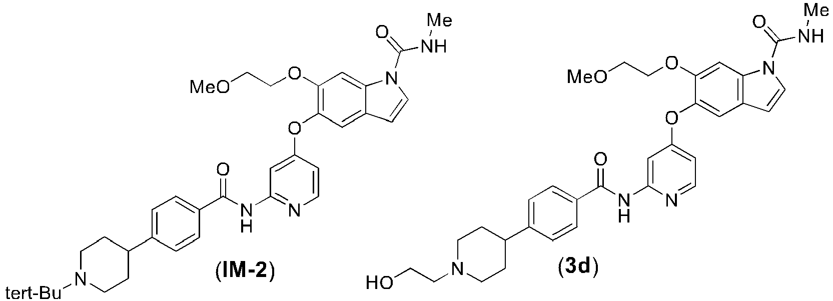
[34] Compound (3d) or a salt thereof, wherein the content of compound (3d) or a salt thereof is 97.0 mass% or greater and the content of compound (IM-2) is 0.15 mass% or lower, 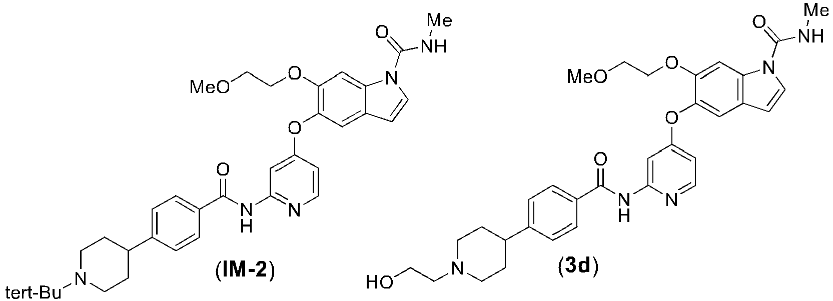
[35] Compound (3d) or a salt thereof, wherein the content of compound (3d) or a salt thereof is 97.0 mass% or greater and the content of compound (3c) is 0.30 mass% or lower, 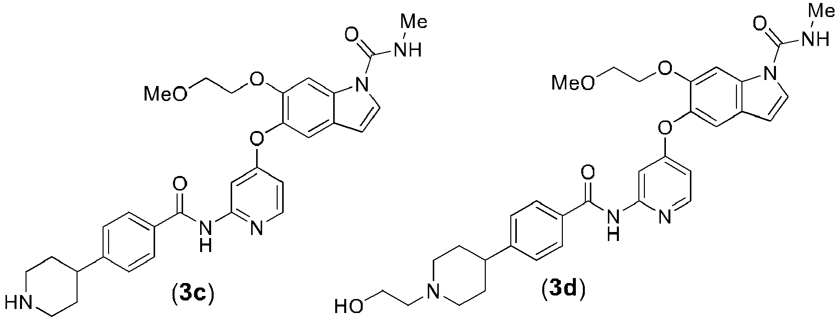
[36] Compound (3d) or a salt thereof, wherein the content of compound (3d) or a salt thereof is 97.0 mass% or greater and the content of compound (IM-3) is 0.30 mass% or lower, 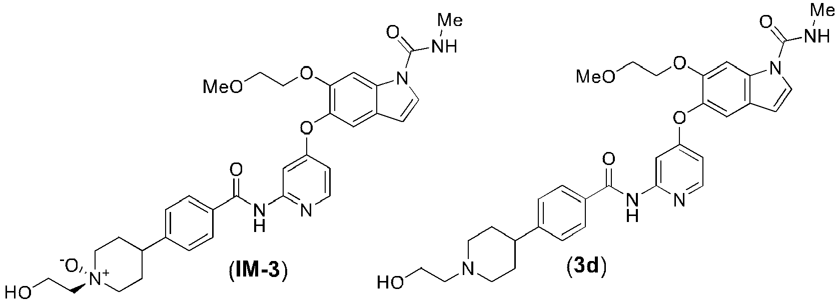
[37] Compound (3d) or a salt thereof, wherein the content of compound (3d) or a salt thereof is 97.0 mass% or greater and the content of compound (IM-3) is 0.15 mass% or lower, 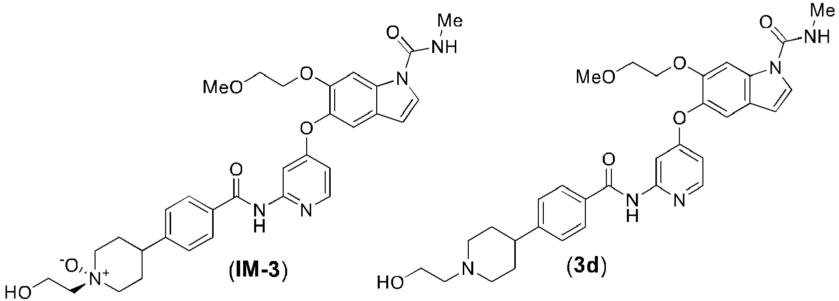
[38] Compound (3d) or a salt thereof, wherein the content of compound (3d) or a salt thereof is 97.0 mass% or greater and the content of compound (2i) is 0.15 mass% or lower, 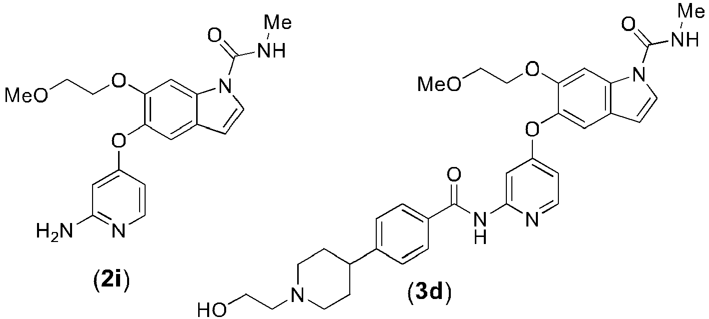
[39] Compound (3d) or a salt thereof, wherein the content of compound (3d) or a salt thereof is 97.0 mass% or greater and the content of compound (IM-4) is 0.15 mass% or lower, 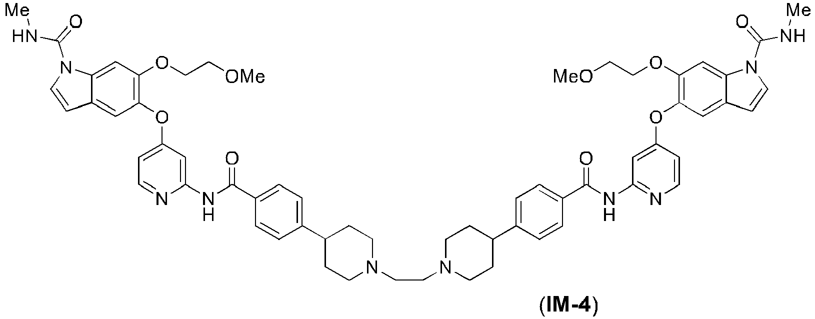 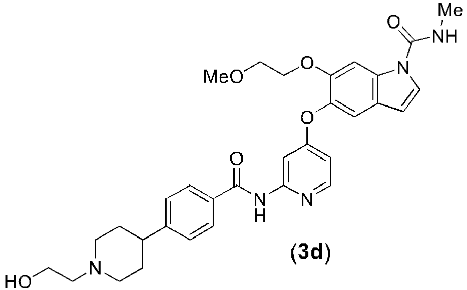
[40] Compound (3d) or a salt thereof, wherein the content of compound (3d) or a salt thereof is 97.0 mass% or greater and the content of compound (IM-6) is 0.15 mass% or lower, 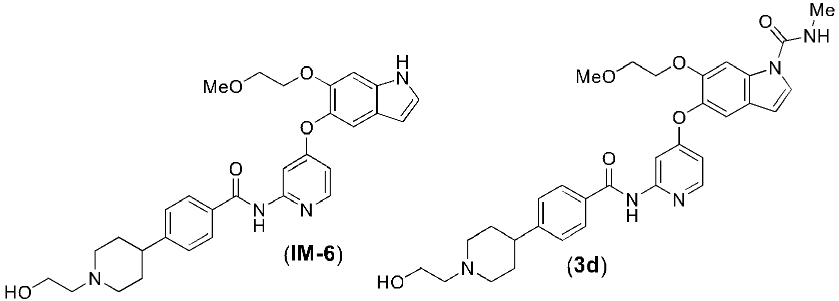
[41] Compound (3d) or a salt thereof, wherein the content of compound (3d) or a salt thereof is 97.0 mass% or greater and the content of compound (IM-8) is 0.15 mass% or lower, 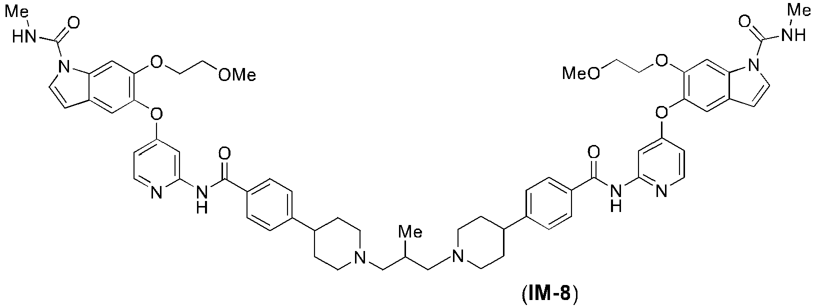 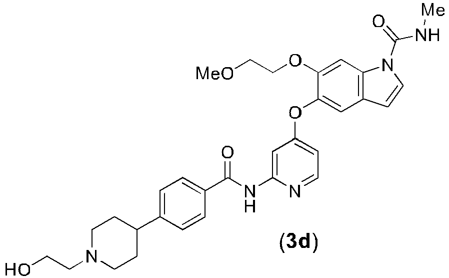
[42] Compound (3d) or a salt thereof, wherein the content of compound (3d) or a salt thereof is 97.0 mass% or greater, the content of compound (IM-7) is 0.48 mass% or lower, and the content of all analogs is 2.0 mass% or lower, 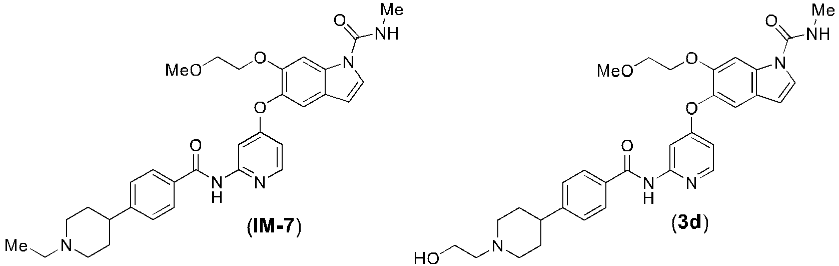
[43] Compound (3d) or a salt thereof, wherein the content of compound (3d) or a salt thereof is 97.0 mass% or greater, the content of compound (IM-5) is 0.40 mass% or lower, and the content of all analogs is 2.0 mass% or lower, 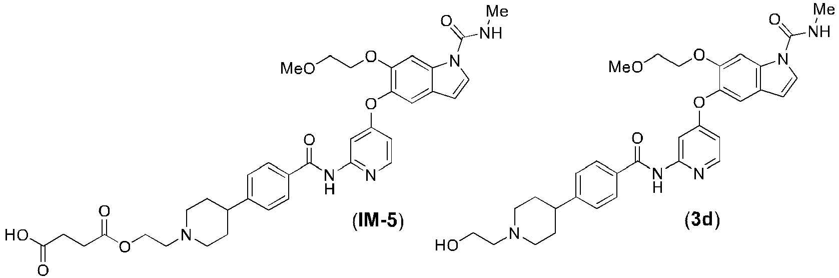
[44] Compound (3d) or a salt thereof, wherein the content of compound (3d) or a salt thereof is 97.0 mass% or greater, the content of compound (IM-2) is 0.30 mass% or lower, and the content of all analogs is 2.0 mass% or lower, 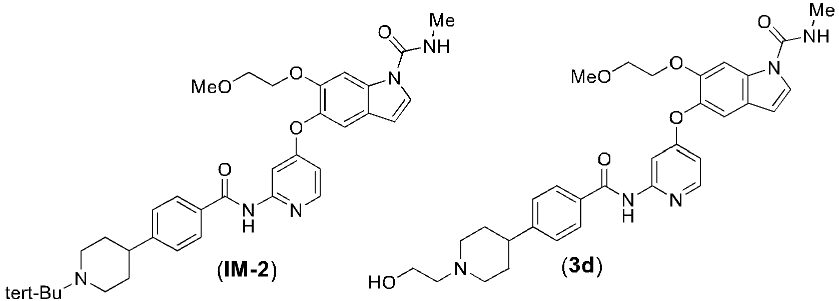
[45] Compound (3d) or a salt thereof, wherein the content of compound (3d) or a salt thereof is 97.0 mass% or greater, the content of compound (IM-2) is 0.15 mass% or lower, and the content of all analogs is 2.0 mass% or lower, 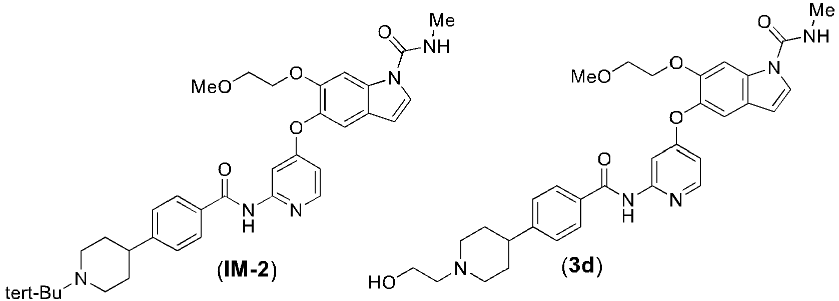
[46] Compound (3d) or a salt thereof, wherein the content of compound (3d) or a salt thereof is 97.0 mass% or greater, the content of compound (3c) is 0.30 mass% or lower, and the content of all analogs is 2.0 mass% or lower, 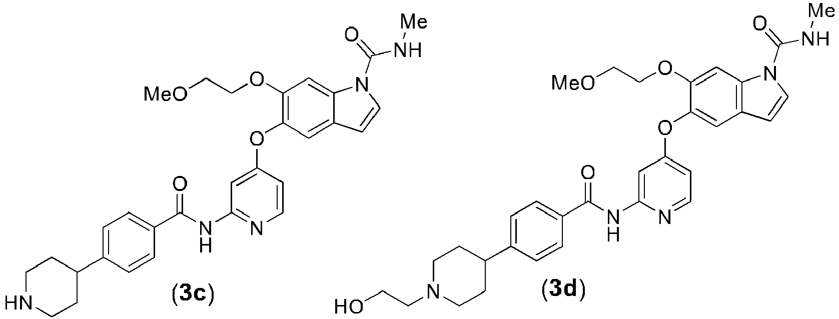
[47] Compound (3d) or a salt thereof, wherein the content of compound (3d) or a salt thereof is 97.0 mass% or greater, the content of compound (IM-3) is 0.30 mass% or lower, and the content of all analogs is 2.0 mass% or lower, 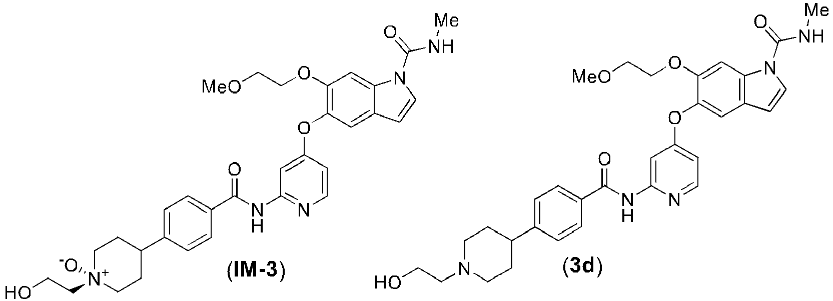
[48] Compound (3d) or a salt thereof, wherein the content of compound (3d) or a salt thereof is 97.0 mass% or greater, the content of compound (IM-3) is 0.15 mass% or lower, and the content of all analogs is 2.0 mass% or lower, 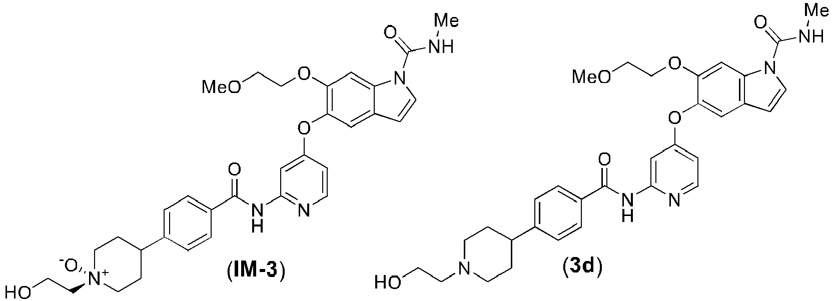
[49] Compound (3d) or a salt thereof, wherein the content of compound (3d) or a salt thereof is 97.0 mass% or greater, the content of compound (2i) is 0.15 mass% or lower, and the content of all analogs is 2.0 mass% or lower, 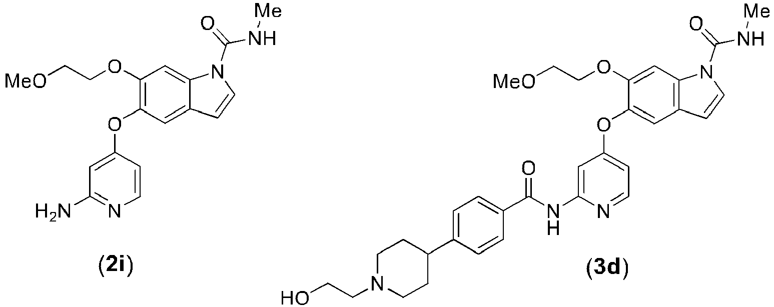
[50] Compound (3d) or a salt thereof, wherein the content of compound (3d) or a salt thereof is 97.0 mass% or greater, the content of compound (IM-4) is 0.15 mass% or lower, and the content of all analogs is 2.0 mass% or lower, 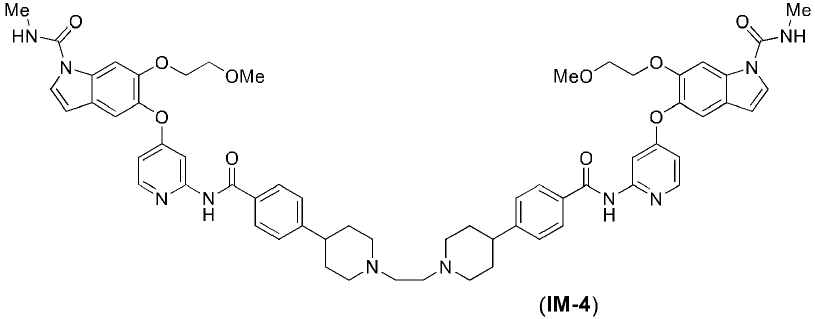 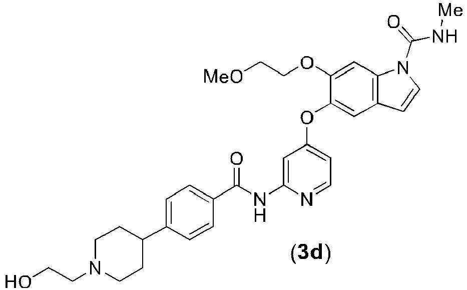
[51] Compound (3d) or a salt thereof, wherein the content of compound (3d) or a salt thereof is 97.0 mass% or greater, the content of compound (IM-6) is 0.15 mass% or lower, and the content of all analogs is 2.0 mass% or lower, 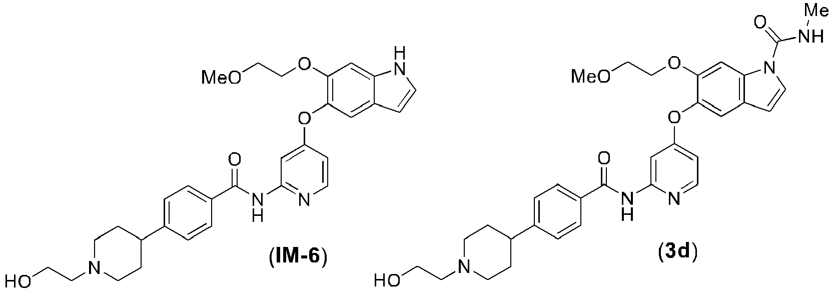
[52] Compound (3d) or a salt thereof, wherein the content of compound (3d) or a salt thereof is 97.0 mass% or greater, the content of compound (IM-8) is 0.15 mass% or lower, and the content of all analogs is 2.0 mass% or lower, 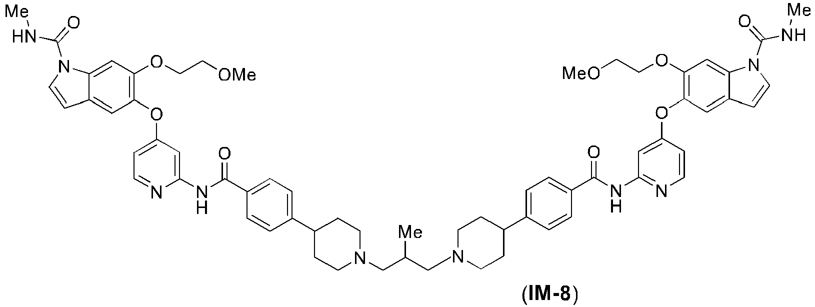 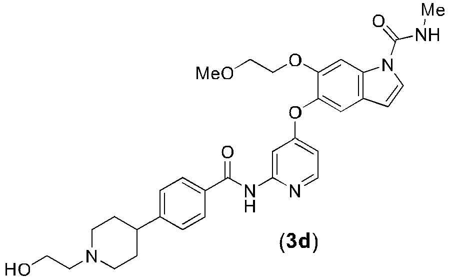
[53] A 1.5 succinate of compound (3d), wherein the succinic acid content in the 1.5 succinate of compound (3d) is 20.8 mass% to 25.5 mass%, and 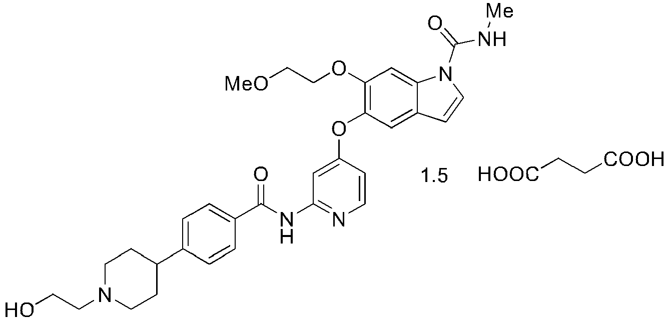
[54] A 1.5 succinate of compound (3d), wherein the succinic acid content in the 1.5 succinate of compound (3d) is 21.9 mass% to 24.3 mass%.

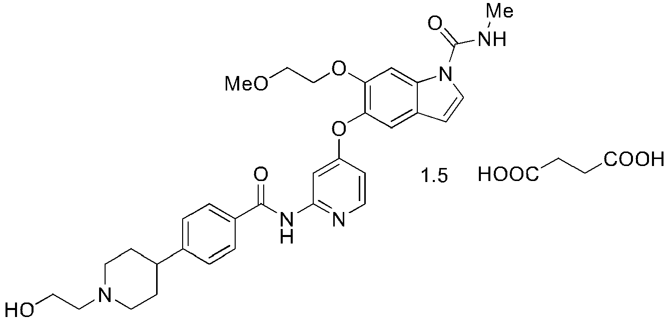

### Advantageous Effects of Invention

According to the present invention it is possible to provide a production process whereby high-quality E7090 with low content of all analogs can be synthesized at higher yield and at greater working efficiency, as well as the high-quality E7090.

### Brief Description of Drawings

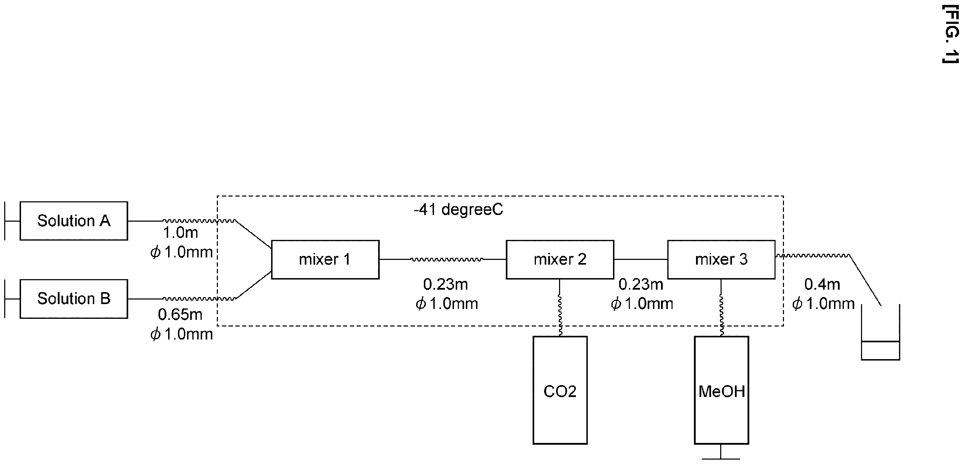

Fig. 1 is a flow chart for flow reaction using the microreactor apparatus of Example 3.

### Description of Embodiments

The definitions of the symbols and terms used throughout the present specification will now be explained, prior to a more detailed description of the invention.

The term "base" as used herein may refer to an inorganic base such as lithium hydroxide, sodium hydroxide, potassium hydroxide, lithium carbonate, sodium carbonate, potassium carbonate, potassium tert-butoxide, sodium tert-butoxide, sodium hydrogencarbonate, potassium hydrogencarbonate or cesium carbonate; an organometallic reagent such as butyllithium, methyllithium, lithium bis(trimethylsilyl)amide, sodium bis(trimethylsilyl)amide or potassium bis(trimethylsilyl)amide; a hydride such as lithium hydride, sodium hydride or potassium hydride; a heterocyclic compound such as imidazole, pyridine, dimethylpyridine, trimethylpyridine or N,N-dimethylaminopyridine; or an organic amine such as triethylamine, N,N-diisopropylethylamine or diazabicycloundecene.

The term "compound" as used herein includes anhydrides, hydrates and solvates. The term "compound (3d)" as used herein has the same meaning as "a compound represented by formula (3d)".

The term "analog" means an organic compound of known or unknown structure other than compound (3d) or a salt thereof, such as a starting substance, intermediate or reagent in the production process for compound (3d) or a salt thereof; an organic compound resulting as a by-product from a starting substance, intermediate or reagent, or resulting from decomposition of a starting substance, intermediate or reagent, in the production process for compound (3d) or a salt thereof; or an organic compound produced by decomposition of compound (3d) or a salt thereof during storage.

Examples of organic compounds of known structure include compound (2i), compound (3c), compound (IM-2), compound (IM-3), compound (IM-4), compound (IM-5), compound (IM-6), compound (IM-7) and compound (IM-8).

The term "all analogs" means all of the analogs of known structure and unknown structure contained by compound (3d) or a salt thereof.

The term "content of all analogs" refers to the total amount (mass%) of all analogs detected by a prescribed test method, such as Test Example 1, for example.

Therefore, the term "compound or a salt thereof' as used herein also includes the concept of "composition", since it may include analogs. The term "composition" means that the "compound or a salt thereof' includes other analogs of the compound or a salt thereof, and thus differs from the term "pharmaceutical composition" defined below. As one embodiment, the compound or a salt thereof is present at 90 mass% or greater, and may include analogs.

The term "pharmaceutical composition" as used herein, refers to a composition comprising a compound or a salt thereof having pharmacological action, and a pharmaceutically acceptable carrier. For example, the compound or a salt thereof with pharmacological action may be compound (3d) or a salt thereof.

The term "salt" as used herein refers to, for example, inorganic acid salts (such as sulfates, nitrates, perchlorates, phosphates, carbonates, bicarbonates, hydrofluorides, hydrochlorides, hydrobromides and hydroiodides), organic carboxylic acid salts (such as acetates, oxalates, maleates, fumarates, succinates, tartrates and citrates), organic sulfonic acid salts (such as methanesulfonates, trifluoromethanesulfonates, ethanesulfonates, benzenesulfonates, toluenesulfonates and camphorsulfonates), and salts of acidic amino acids (such as aspartates and glutamates).

According to one embodiment it is possible to provide the compound described herein as a salt, such as a pharmaceutically acceptable salt. The term "pharmaceutically acceptable salt" refers to a salt that retains the desired biological activity of the parent compound, and does not introduce any undesirable toxicological effects. Specific examples of pharmaceutically acceptable salts include inorganic acid salts (such as sulfates, nitrates, perchlorates, phosphates, carbonates, bicarbonates, hydrofluorides, hydrochlorides, hydrobromides and hydroiodides), organic carboxylic acid salts (such as acetates, oxalates, maleates, fumarates, succinates, tartrates and citrates), organic sulfonic acid salts (such as methanesulfonates, trifluoromethanesulfonates, ethanesulfonates, benzenesulfonates, toluenesulfonates and camphorsulfonates), and amino acid salts (such as aspartates and glutamates), quaternary amine salts, and alkali metal salts (sodium salts and potassium salts) or alkaline earth metal salts (magnesium salts and calcium salts).

There are no particular restrictions on salts of compound (3d), and examples include inorganic acid salts, organic acid salts and acidic amino acid salts.

There are also no particular restrictions on salts of compound (2i), and examples include inorganic acid salts, organic acid salts and acidic amino acid salts.

Compound (3d) or a salt thereof may also be an anhydride, hydrate or solvate.

The present invention also encompasses isotope-labeled compounds of the compounds listed in the present specification, and production processes using them. An isotope-labeled compound is the same as any of the compounds mentioned herein, except that one or more atoms are replaced with an atom having a different atomic mass or mass number than the atomic mass or mass number usually observed in nature. Examples of isotopes that may be incorporated into compounds of the invention include isotopes of hydrogen, carbon, nitrogen, oxygen, fluorine, chlorine, phosphorus, sulfur and iodine, and specifically ²H, ³H, ¹¹C, ¹⁴C, ¹³N, ¹⁵O, ¹⁸F, ³²P, ³⁵S, ¹²³I and ¹²⁵I. Compounds of the invention that include these isotopes and/or other isotopes, as well as their pharmaceutically acceptable derivatives (such as salts), are also within the scope of the present Claims.

Isotope-labeled compounds of the invention, such as compounds incorporating radioactive isotopes such as ³H and/or ¹⁴C, may be useful for tissue distribution assays of drugs and/or substrates. The isotopes ³H and ¹⁴C are considered useful because of their ease of preparation and detection. The isotopes ¹¹C and ¹⁸F are considered useful for PET (Positron Emission Tomography), while the isotope ¹²⁵I is considered useful for SPECT (Single Photon Emission Computed Tomography), and all of their isotopes are useful for brain imaging. Substitution of ²H and the like with heavier isotopes affords advantages in certain types of treatment, such as a longer *in vivo* half-life and lower required dosages due to higher metabolic stability, and are therefore considered useful under some circumstances.

The production process of the invention will now be explained in greater detail.

### Production process for compound (3d) or a salt thereof

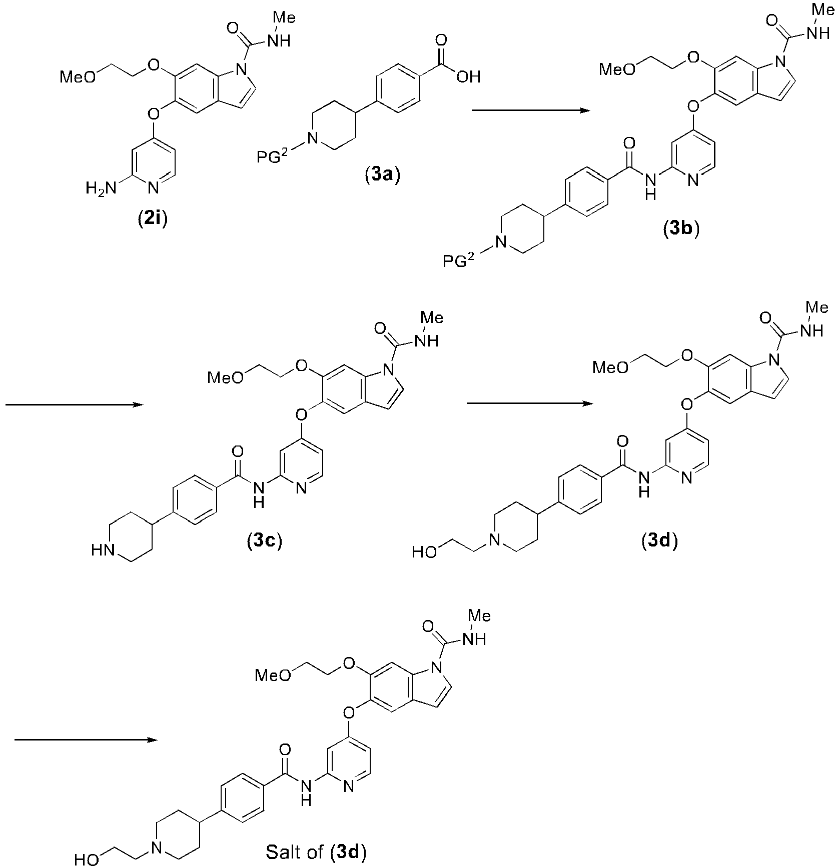

Step a) is a step in which compound (2i) or a salt thereof and compound (3a) are reacted in the presence of a condensation agent to obtain compound (3b).

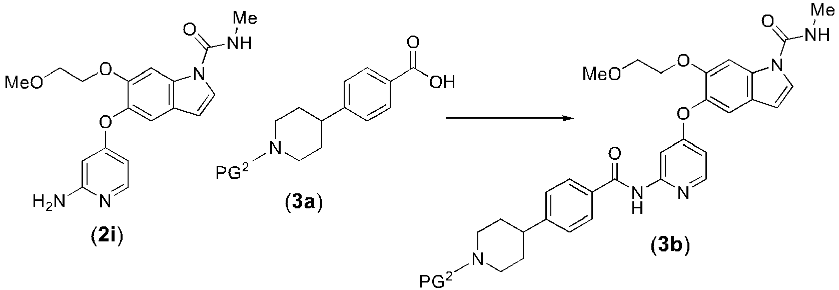

Compound (2i) may also be a salt thereof. When a salt of compound (2i) is used, it is preferably a methanesulfonate.

The protecting group for the piperidyl group of compound (3a) may be a tert-butoxycarbonyl, benzyloxycarbonyl or allyloxycarbonyl group. It is preferably a tert-butoxycarbonyl group.

The condensation agent may be diethyl phosphate cyanide (DEPC), 1-ethyl-3-(3-dimethylaminopropyl)carbodiimide hydrochloride (WSC-HCl/EDC-HCl), dicyclohexyl carbodiimide (DCC), 2-chloro-N-methylpyridinium iodide (CMPI), 2,4,6-trichlorobenzoyl chloride, propylphosphonic anhydride (cyclic trimer), (benzotriazole-1 -yloxy)tripyrrolidinophosphonium hexafluorophosphate (PyBOP), O-(7-azabenzotriazol-1-yl)-N,N,N',N'-tetramethyluronium hexafluorophosphate (HATU) or 4-(4,6-dimethoxy-1,3,5-triazin-2-yl)-4-methylmorpholinium chloride n-hydrate (DMT-MM). It is preferably 1-ethyl-3-(3-dimethylaminopropyl)carbodiimide hydrochloride (WSC-HCl/EDC-HCl). The condensation agent may be used at 1.0 to 3.0 equivalents with respect to compound (2i). It is preferably used at 1.5 to 2.5 equivalents.

When compound (3a) is 4-(1-(*tert*-butoxycarbonyl)piperidin-4-yl)benzoic acid (3a), compound (3a) may be used at 1.0 to 2.0 equivalents with respect to compound (2i). It is preferably used at 1.1 to 1.3 equivalents.

The base used in step a) may be triethylamine, N,N-dimethylaminopyridine (DMAP), 1-methylimidazole, diisopropylethylamine or 1-methylpiperazine. Preferably, N,N-dimethylaminopyridine (DMAP) is used. The base may be used at 0.1 to 5 equivalents with respect to compound (2i). It is preferably used at 1 to 3 equivalents.

The activating agent used in step a) may be N,N-dimethylaminopyridine (DMAP) or 1-hydroxybenzotriazole (HOBt). Preferably, N,N-dimethylaminopyridine (DMAP) is used. The activating agent may be used at 1.0 to 3.0 equivalents with respect to compound (2i). It is preferably used at 1.5 to 2.5 equivalents.

The solvent is not particularly restricted so long as it dissolves the starting substance and does not interfere with the reaction, and for example, it may be DMF, DMSO, acetonitrile or 1,2-dimethoxyethane. The solvent is preferably 1,2-dimethoxyethane.

The reaction temperature will generally differ depending on the starting substances, solvent and other reagents used in the reaction, but it is preferably 20°C to 80°C. The temperature is preferably 40°C to 70°C.

In step a), 1-methylpiperazine, 1-ethylpiperazine or water may be used as an imide group-cleaving reagent. It is preferred to use 1-methylpiperazine. The imide group-cleaving reagent may be used at 0.1 to 1.0 equivalents and preferably 0.2 to 0.4 equivalents with respect to compound (2j).

The reaction temperature for imide group cleavage will generally differ depending on the starting substances, solvent and other reagents used in the reaction, but it is preferably 20°C to 80°C. The temperature is preferably 40°C to 70°C.

Step b) is a step in which PG² is removed from compound (3b) to obtain compound (3c).

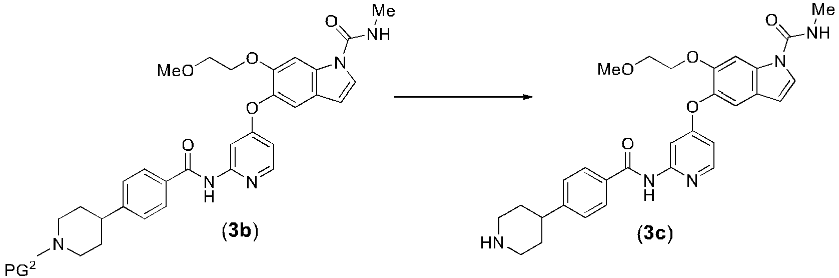

In step b), the deprotection conditions used may be those suited for the protecting group. For example, deprotection may be carried out under acidic conditions in the case of a tert-butoxycarbonyl group, under basic conditions or reducing conditions in the case of a benzyloxycarbonyl group, using a secondary amine such as piperidine in the case of a 9-fluorenylmethyloxycarbonyl group, or using a thiol under basic conditions in the case of a 2-nitrobenzenesulfonyl group.

When the protecting group is a tert-butoxycarbonyl group, the acid may be trifluoroacetic acid, formic acid, sulfuric acid, hydrochloric acid, phosphoric acid, potassium hydrogensulfate or methanesulfonic acid, for example. Formic acid and hydrochloric acid are preferred.

The solvent is not particularly restricted so long as it dissolves the starting substance and does not interfere with the reaction, and for example, it may be methanol, ethanol, 2-propanol, 1,2-dimethoxyethane, tetrahydrofuran or dioxane, or a mixed solvent of an organic solvent thereof and water, or no solvent may be used. The solvent is preferably 2-propanol.

The reaction temperature will generally differ depending on the starting substances, solvent and other reagents used in the reaction, but it is preferably room temperature to 70°C, and more preferably 20°C to 50°C.

Step c) is a step in which compound (3c) and a hydroxyethylating agent are reacted to obtain compound (3d).

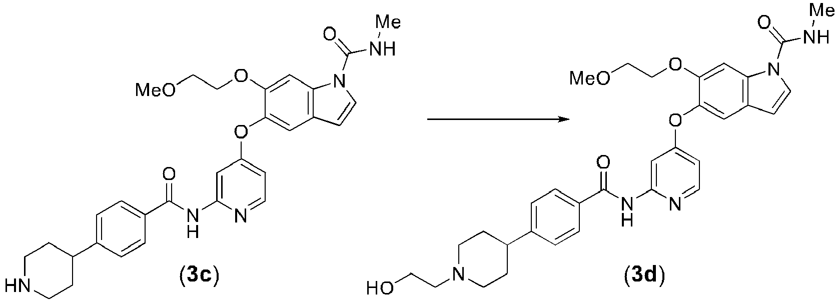

The hydroxyethylating agent may be 1,4-dioxane-2,5-diol, for example. When the hydroxyethylating agent is 1,4-dioxane-2,5-diol, it may be used at 1.0 to 3.0 equivalents with respect to compound (3c). It is preferably used at 0.6 to 1.0 equivalents.

When the hydroxy ethylating agent is 1,4-dioxane-2,5-diol, a reducing agent is preferably used in step c). The reducing agent may be sodium borohydroxide, sodium triacetoxyborohydride, sodium borohydride or sodium cyanoborohydride. It is preferably sodium triacetoxyborohydride. The amount of the reducing agent may be 0.5 to 4.0 equivalents with respect to compound (3c). It is preferably used at 2.0 to 4.0 equivalents.

The reaction solvent is not particularly restricted so long as it dissolves the starting substance and does not interfere with the reaction, and for example, it may be dimethoxyethane, tetrahydrofuran, acetonitrile, 1-butanol, ethanol, methanol, or a mixture of these. The solvent is preferably methanol.

The reaction temperature will generally differ depending on the starting substances, solvent and other reagents used in the reaction, but it is preferably -35°C to room temperature. The temperature is preferably -15°C to 10°C.

Step d) is a step in which compound (3d) is converted to a salt.

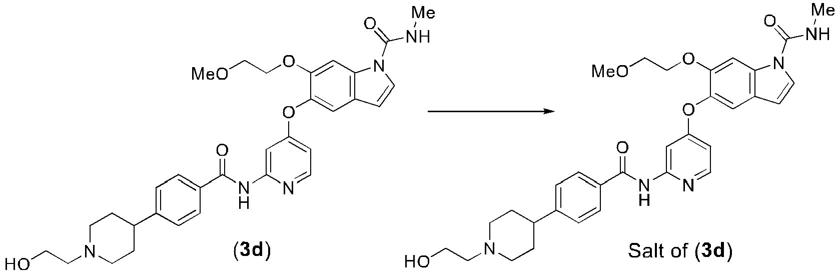

Compound (3d) can be converted to a pharmaceutically acceptable salt by the process described in PTL 2, for example.

Compound (3d) or a salt thereof to be used for crystallization may be in any prepared form such as a solvate, hydrate or anhydride, and it may be either amorphous or crystalline (including multiple polymorphic crystals), or mixtures of these.

The acid may be used at 1.0 to 3.0 equivalents with respect to compound (3d). Succinic acid is used at 1.7 to 2.0 equivalents with respect to compound (3d).

The solvent to be used for crystallization may be, for example, an alcohol-based solvent such as methanol, ethanol, 1-propanol or 2-propanol; acetonitrile; an amide-based solvent such as N,N-dimethylformamide; an ester-based solvent such as ethyl acetate; a saturated hydrocarbon-based solvent such as hexane or heptane; a ketone-based solvent such as acetone or 2-butanone, an ether-based solvent such as *tert*-butyl methyl ether, or water. These solvents may be used alone, or two or more different ones may be used in admixture. For crystallization of a succinate, it is preferred to use a mixed solvent of 2-propanol and water.

The amount of solvent used may be appropriately selected, with the lower limit as an amount which allows compound (3d) or a salt thereof to dissolve by heating or an amount that allows stirring of the suspension, and the upper limit as an amount which does not notably reduce the crystal yield.

For crystallization, seed crystals may be added (desired crystals of a salt of compound (3d)), but they do not need to be added. The temperature for adding seed crystals is not particularly restricted but is preferably 0 to 60°C. The seed crystals used may be crystals produced by the process described in PTL 2.

The temperature for dissolution of compound (3d) or a salt thereof by heating may be selected as an appropriate temperature that dissolves compound (3d) or a salt thereof in the solvent used, but it is preferably in a range from 30°C to the temperature at which the recrystallization solvent begins to undergo reflux, and it is more preferably 30 to 70°C.

Since quenching can result in inclusion of crystals of different types (polymorphs), the cooling during crystallization is preferably carried out at an appropriate cooling rate considering its effect on the quality and particle sizes of the crystal, and it is preferably cooling at a rate of 5 to 40°C/hour, for example. The cooling rate is more preferably 5 to 25°C/hour, for example.

The final crystallization temperature may be appropriately selected based on the crystal yield and quality, but it is preferably -25 to 30°C.

The formed crystals are separated by an ordinary filtration procedure, and if necessary the filtered crystals are rinsed with a solvent and dried to obtain the desired crystals. The solvent used for rinsing of the crystals may be the same as the crystallization solvent. Preferred solvents are ethanol, acetone, 2-propanol, 2-butanone, ethyl acetate, diethyl ether, *tert*-butyl methyl ether and hexane. These solvents may be used alone, or two or more different ones may be used in admixture.

The crystals separated by the filtration procedure may be appropriately dried by standing under air or a nitrogen stream, or by heating.

The drying time may be appropriately selected as the time at which the residual solvent falls below a prescribed amount, and will depend on the production amount, the drying apparatus and the drying temperature. The drying may be carried out with ventilation or under reduced pressure. The degree of pressure reduction may be appropriately selected depending on the production amount, the drying apparatus and the drying temperature. The obtained crystal may be dried and then left to stand in air if necessary.

Another embodiment of the invention is a pharmaceutical composition comprising "compound (3d) or a salt thereof' or a crystal thereof, and a pharmaceutically acceptable additive. The pharmaceutical composition can be produced by mixing a pharmaceutically acceptable additive with the "compound (3d) or a salt thereof' or a crystal thereof. The pharmaceutical composition of the invention can be produced by a known method, such as the method described in the General Rules for Preparations of the Japanese Pharmacopoeia, 18th Edition.

The pharmaceutical composition of the embodiment may be appropriately administered to a patient as suitable for the dosage form.

The dose of "compound (3d) or a salt thereof' or a crystal thereof according to the invention will differ depending on the severity of symptoms, the patient age, gender and body weight, the form of administration, the type of salt and the specific type of disease, but for most cases it may be 1 mg to 500 mg per day for oral administration to an adult (60 kg body weight), while according to one embodiment it is 10 mg to 300 mg, and according to another embodiment it is 20 mg to 200 mg. It may be administered in 1 to 3 portions per day.

### Examples

The present invention will now be explained in greater detail by Examples. However, the invention is not limited to these Examples. The abbreviations used here throughout are those commonly known to those skilled in the art, and a few are indicated below.

The ¹H-NMR spectra were measured using a BRUCKER AVANCE NEO 400 (400 MHz), BRUCKER AVANCE III 500 (500 MHz), BRUCKER AVANCE 600 (600 MHz) or BRUCKER AVANCE NEO 700 (700 MHz).

The chemical shifts in the proton nuclear magnetic resonance (¹H-NMR) spectra are recorded in δ units (ppm) with respect to tetramethylsilane, and the coupling constants are recorded in Hertz (Hz). The patterns are indicated as s: singlet, d: doublet, br: broad or m: multiplet.

The term "room temperature" in the Examples generally refers to a temperature of between about 10°C and 35°C. The percentage values are mass percentages, unless otherwise specified.

### Example 1: Production of 5-({2-[({4-[1-(2-hydroxyethyl)piperidin-4-yl]phenyl}carbonyl)amino]pyridin-4-yl}oxy)-6-( 2-methoxyethoxy)-N-methyl-1H-indole-1-carboxamide butanedioate (2:3) (E7090)

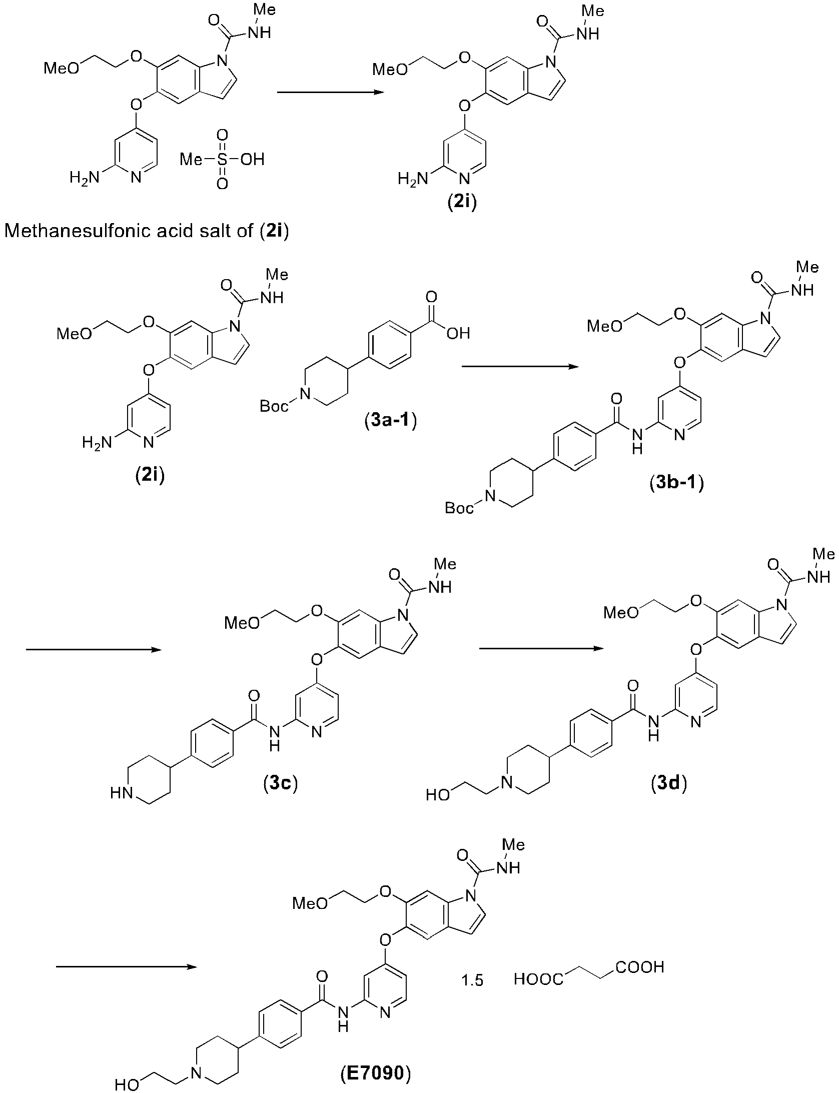

### Production Example 1: Production of 5-((2-aminopyridin-4-yl)oxy)-6-(2-methoxyethoxy)-N-methyl-1H-indole-1-carboxamide (2i)

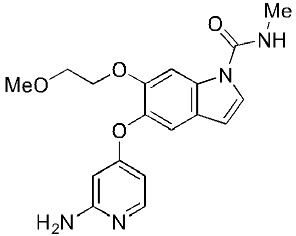

An aqueous solution of 1 N sodium hydroxide (2.71 kg caustic soda flake, 67.7 mol, 1.54 eq., 67.7 kg water) was added to a suspension of 5-((2-aminopyridin-4-yl)oxy)-6-(2-methoxyethoxy)-N-methyl-1H-indole-1-carboxamide methanesulfonate (19.9 kg, 44.0 mol) in tetrahydrofuran (159.2 kg) under a nitrogen atmosphere, and the mixture was stirred at 25°C for 30 minutes. Isopropyl acetate (156.2 kg) was added to the reaction mixture prior to liquid separation, and then the organic layer was rinsed with 5% brine (2.99 kg salt, 56.7 kg water). The obtained organic layer was rinsed with water (59.7 kg) and then clarified by filtration and washed in with isopropyl acetate (8.7 kg). It was subsequently concentrated under reduced pressure to 100 L at 40°C or lower, and further subjected to azeotropic distillation 4 times with acetonitrile (78.2 kg). Acetonitrile (15.6 kg) was added to the concentrate and the mixture was stirred for 1 hour at 48°C. The suspension was cooled to 0°C and then filtered and rinsed with acetonitrile (23.5 kg). The obtained crystals were dried under reduced pressure at 50°C or lower to obtain 13.91 kg of the title compound.
¹H NMR Spectrum (DMSO-d₆) δ (ppm): 2.83 (3H, d, J=4.4 Hz), 3.18 (3H, s), 3.50-3.54 (2H, m), 4.04-4.08 (2H, m), 5.69 (1H, d, J=1.8 Hz), 5.76 (2H, s), 6.09 (1H, dd, J=5.7, 2.2 Hz), 6.59 (1H, d, J=3.5 Hz), 7.33 (1H, s), 7.71-7.74 (2H, m), 8.03 (1H, s), 8.10-8.14 (1H, m)

### Production Example 1-2: Production of 5-((2-aminopvridin-4-yl)oxy)-6-(2-methoxyethoxy)-N-methyl-1H-indole-1-carboxamide (2i)

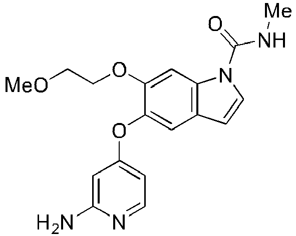

An aqueous solution of 1 N sodium hydroxide (5.5 kg caustic soda flake, 137.5 mol, 1.70 eq., 138 kg water) was added to a suspension of 5-((2-aminopyridin-4-yl)oxy)-6-(2-methoxyethoxy)-N-methyl-1H-indole-1-carboxamide methanesulfonate (36.6 kg, 80.9 mol) in tetrahydrofuran (292.5 kg) under a nitrogen atmosphere, and the mixture was stirred at 20°C for 30 minutes. Isopropyl acetate (287 kg) was added to the reaction mixture prior to liquid separation, and then the organic layer was rinsed with 5% brine (5.5 kg salt, 104 kg water). The obtained organic layer was rinsed with water (110 L) and then clarified by filtration and washed in with isopropyl acetate (47.9 kg). It was subsequently concentrated under reduced pressure to 184 L at 40°C or lower, and further subjected to azeotropic distillation 4 times with acetonitrile (144 kg). Acetonitrile (28.8 kg) was added to the concentrate and the mixture was stirred for 1 hour at 45 to 46°C. The suspension was cooled to 2°C and then filtered and rinsed with acetonitrile (43.2 kg). The obtained crystals were dried under reduced pressure at 50°C or lower to obtain 25.92 kg of the title compound.

### Production Example 2: Production of tert-butyl 4-(4-((4-((6-(2-methoxyethoxy)-1-(methylcarbamoyl)-1H-indol-5-yl)oxy)pyridin-2-yl)carba moyl)phenyl)piperidine-1-carboxylate (3b-1)

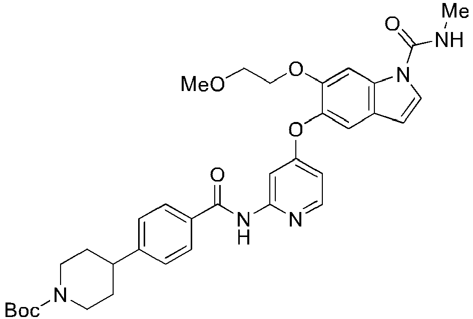

A suspension of 5-((2-aminopyridin-4-yl)oxy)-6-(2-methoxyethoxy)-N-methyl-1H-indole-1-carboxamide (15.0 kg, 42.1 mol), 4-(1-(*tert*-butoxycarbonyl)piperidin-4-yl)benzoic acid (15.4 kg, 50.5 mol, 1.2 eq.), N,N-dimethyl-4-aminopyridine (10.3 kg, 84.2 mol, 2.0 eq.) and 1-ethyl-3-(3-dimethylaminopropyl)carbodiimide hydrochloride (16.1 kg, 84.2 mol, 2.0 eq.) in 1,2-dimethoxyethane (105 L) was stirred at 55°C for 1 hour under a nitrogen atmosphere. Upon completion of the reaction, 1-methylpiperazine (1.3 kg, 12.6 mol, 0.30 eq.) was added and the mixture was stirred at 55°C for 1 hour. Upon completion of the reaction, the reaction mixture was cooled to an internal temperature of 10 to 15°C, and then ethyl acetate (225 L) and 2 N hydrochloric acid (17.7 kg concentrated hydrochloric acid, 75 L water) were added prior to liquid separation. To the obtained organic layer was added 5% sodium bicarbonate water (3.8 kg sodium bicarbonate, 71.3 kg water) prior to liquid separation. The obtained organic layer was concentrated under reduced pressure to 115 L at an external temperature of 50°C, and ethyl acetate (13 L) was added to adjust to 128 L. Ethyl acetate (38 L) was added to the concentrate and the mixture was stirred for 2 hours at 25°C, after which *n*-heptane (150 L) was added dropwise and stirring was continued at the same temperature. The mixture was filtered and rinsed with a mixture of ethyl acetate and *n*-heptane (ethyl acetate/n-heptane = 2.5/2.5vol., 76 L). The obtained crystals were dried under reduced pressure at 50°C to obtain 24.4 kg of the title compound.
¹H NMR Spectrum (DMSO-d₆) δ (ppm): 1.40 (9H, s), 1.45-1.53 (2H, m), 1.75 (2H, br d, J=13.4 Hz), 2.70-2.90 (6H, m), 3.11 (3H, s), 3.46-3.49 (2H, m), 4.00-4.11 (4H, m), 6.62 (1H, d, J=3.6 Hz), 6.66 (1H, dd, J=5.7, 2.3 Hz), 7.33 (2H, d, J=8.3 Hz), 7.43 (1H, s), 7.68 (1H, d, J=2.3 Hz), 7.77 (1H, d, J=3.6 Hz), 7.89 (2H, d, J=8.3 Hz), 8.07 (1H, s), 8.14 (1H, q, J=4.5 Hz), 8.18 (1H, d, J=5.7 Hz), 10.61 (1H, s)

### Production Example 2-2: Production of tert-butyl 4-(4-((4-((6-(2-methoxyethoxy)-1-(methylcarbamoyl)-1H-indol-5-yl)oxy)pyridin-2-yl)carba moyl)phenyl)piperidine-1-carboxylate (3b-1)

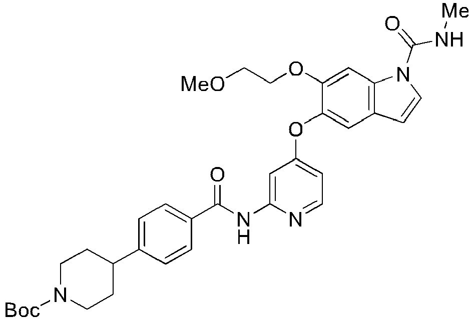

Triethylamine (11.3 kg, 111.7 mol, 2.0 eq.) was added to a suspension of 5-((2-aminopyridin-4-yl)oxy)-6-(2-methoxyethoxy)-N-methyl-1H-indole-1-carboxamide methanesulfonate (26.0 kg, 76.5% content, 19.9 kg, 55.8 mol as free form), 4-(1-(*tert*-butoxycarbonyl)piperidin-4-yl)benzoic acid (20.5 kg, 67.0 mol, 1.2 eq.), N,N-dimethyl-4-aminopyridine (13.6 kg, 111.7 mol, 2.0 eq.) and 1-ethyl-3-(3-dimethylaminopropyl)carbodiimide hydrochloride (21.4 kg, 111.7 mol, 2.0 eq.) in 1,2-dimethoxyethane (139 L) under a nitrogen atmosphere, and the mixture was stirred at 55°C for 1 hour. Upon completion of the reaction, 1-methylpiperazine (1.7 kg, 16.8 mol, 0.3 eq.) was added and the mixture was stirred at 55°C for 1 hour. Upon completion of the reaction, the reaction mixture was cooled to an internal temperature of 10 to 15°C, and then ethyl acetate (299 L) and 2 N hydrochloric acid (35.3 kg concentrated hydrochloric acid, 150.0 L water) were added prior to liquid separation. To the obtained organic layer was added 5% sodium bicarbonate water (6.0 kg sodium bicarbonate, 113.4 kg water) prior to liquid separation. The obtained organic layer was concentrated under reduced pressure to 165 L at an external temperature of 50°C, and ethyl acetate (4 L) was added to adjust to 169 L. Ethyl acetate (50 L) was added to the concentrate and the mixture was stirred for 2 hours at 30°C, after which *n*-heptane (199 L) was added dropwise and stirring was continued at the same temperature. The mixture was filtered and rinsed with a mixture of ethyl acetate and *n*-heptane (ethyl acetate/*n*-heptane = 2.5/2.5vol., 100 L). The obtained crystals were dried under reduced pressure at 50°C to obtain 35.0 kg of the title compound.

### Production Example 2-3: Production of tert-butyl 4-(4-((4-((6-(2-methoxyethoxy)-1-(methylcarbamoyl)-1H-indol-5-yl)oxy)pyridin-2-yl)carba moyl)phenyl)piperidine-1-carboxylate (3b-1)

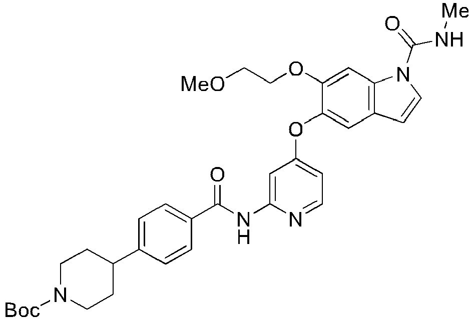

A suspension of 5-((2-aminopyridin-4-yl)oxy)-6-(2-methoxyethoxy)-N-methyl-1H-indole-1-carboxamide (24.0 kg, 67.3 mol), 4-(1-(*tert*-butoxycarbonyl)piperidin-4-yl)benzoic acid (24.7 kg, 80.9 mol), N,N-dimethyl-4-aminopyridine (16.5 kg, 135.1 mol) and 1-ethyl-3-(3-dimethylaminopropyl)carbodiimide hydrochloride (25.8 kg, 134.6 mol) in 1,2-dimethoxyethane (168 L) was stirred at 57°C for 2 hours under a nitrogen atmosphere. To the mixture was added 1-methylpiperazine (2.0 kg, 20.2 mol), and the resulting mixture was stirred at 57°C for 2 hours. The reaction mixture was cooled to an internal temperature of 10 to 15°C, and then ethyl acetate (360 L), water (86 L) and 5 N hydrochloric acid (62.2 kg) were added prior to liquid separation. To the organic layer was added 5% sodium bicarbonate water (6.0 kg sodium bicarbonate, 114 L water) prior to liquid separation. The obtained organic layer was concentrated under reduced pressure to 200 L at an external temperature of 50°C, and ethyl acetate (4 L) was added to adjust to 204 L. Ethyl acetate (60 L) was added to the concentrate and the mixture was stirred for 1 hour at 60°C, and then cooled to an internal temperature of 25°C and stirred for 3 hours. After adding n-heptane (240 L) dropwise, the mixture was filtered and rinsed with a mixture of ethyl acetate and *n*-heptane (ethyl acetate/*n*-heptane = 2.5/2.5 vol., 120 L). The obtained crystals were dried under reduced pressure at 50°C to obtain 41.2 kg of the title compound.

### Production Example 3: Production of 6-(2-methoxyethoxy)-N-methyl-5-{[2-({[4-(piperidinA-yl)phenyl]carbon}amino)pyridin-4-yl]oxy}-1H-indole-1-carboxamide (3c)

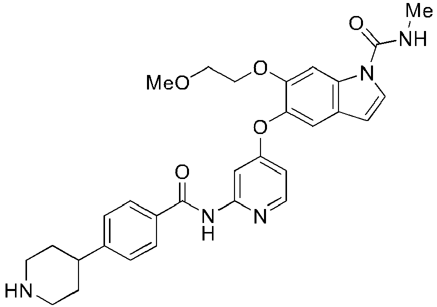

To a suspension of *tert-butyl* 4-(4-((4-((6-(2-methoxyethoxy)-1-(methylcarbamoyl)-1H-indol-5-yl)oxy)pyridin-2-yl)carba moyl)phenyl)piperidine-1-carboxylate (24.4 kg, 37.9 mol) in 2-propanol (49 L) was added 5 N hydrochloric acid (79.1 kg) under a nitrogen atmosphere, and the mixture was stirred at 35°C for 2 hours. Upon completion of the reaction, the reaction mixture was cooled to an internal temperature of 0 to 5°C, after which water (73 L), tetrahydrofuran (244 L) and a 5 N sodium hydroxide aqueous solution (22.0 kg) were added, and the mixture was stirred at 25°C prior to liquid separation. To the organic layer were then added 5% brine (12.2 kg salt, 109.8 kg water) and toluene (24 L), prior to liquid separation. Ethanol (244 L) was added to the obtained organic layer and the mixture was concentrated under reduced pressure to 122 L at an external temperature of 50°C, after which ethanol (49 L) was added to the concentrate and the mixture was stirred for 1 hour at 45°C. The suspension was cooled to an internal temperature of 3°C and then filtered and rinsed with ethanol (98 L). The obtained crystals were dried under reduced pressure at 50°C to obtain 18.5 kg of the title compound.
¹H NMR Spectrum (DMSO-d₆) δ (ppm): 1.45-1.55 (2H, m), 1.67 (2H, br dd, J=12.1, 1.7 Hz), 2.53-2.58 (2H, m), 2.59-2.65 (1H, m), 2.84 (3H, d, J=4.2 Hz), 2.95-3.02 (2H, m), 3.12 (3H, s), 3.45-3.49 (2H, m), 4.05-4.09 (2H, m), 6.62 (1H, br d, J=3.4 Hz), 6.66 (1H, dd, J=5.7, 2.3 Hz), 7.30 (2H, d, J=8.5 Hz), 7.43 (1H, s), 7.68 (1H, d, J=2.3 Hz), 7.77 (1H, d, J=3.6 Hz), 7.89 (2H, d, J=8.3 Hz), 8.07 (1H, s), 8.12-8.16 (1H, m), 8.18 (1H, d, J=5.7 Hz), 10.59 (1H, br s)

### Production Example 3-2: Production of 6-(2-methoxyethoxy)-N-methyl-5-([2-(([4-(piperidin-4-yl)phenyl]carbonyl}amino)pyridin-4-yl]oxy}-1H-indole-1-carboxamide (3c)

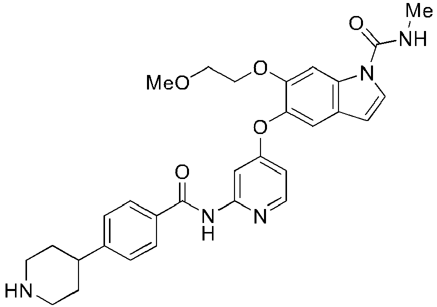

To a suspension of *tert-butyl* 4-(4-((4-((6-(2-methoxyethoxy)-1-(methylcarbamoyl)-1H-indol-5-yl)oxy)pyridin-2-yl)carba moyl)phenyl)piperidine-1-carboxylate (41.2 kg, 64.0 mol) in 2-propanol (82 L) was added 5 N hydrochloric acid (133.5 kg) under a nitrogen atmosphere, and the mixture was stirred at 35°C for 3 hours. The reaction mixture was cooled to an internal temperature of 25°C, and then water (124 L) and toluene (412 L) were added and the mixture was stirred prior to liquid separation. The obtained aqueous layer was cooled to an internal temperature of 0 to 5°C, and then tetrahydrofuran (412 L) and an aqueous 5 N sodium hydroxide solution (37.1 kg sodium hydroxide, 181 L water) were added and the mixture was stirred at 20°C prior to liquid separation. To the organic layer were then added 10% brine (20.6 kg salt, 185 L water) and toluene (41 L), prior to liquid separation. Ethanol (424 L) was added to the obtained organic layer and the mixture was concentrated under reduced pressure to 210 L at an external temperature of 50°C, after which ethanol (82 L) was added to the concentrate and the mixture was stirred at 47°C for 1 hour. The suspension was cooled to an internal temperature of 3°C and then filtered and rinsed with ethanol (165 L). The obtained crystals were dried under reduced pressure at 50°C to obtain 31.3 kg of the title compound.

### Production Example 4: Production of 5-({2-[({4-[1-(2-hydroxyethyl)piperidin-4-yl]phenyl}carbonyl)aminolpyridin-4-yl}oxy)-6-( 2-methoxyethoxy)-N-methyl-1H-indole-1-carboxamide (3d)

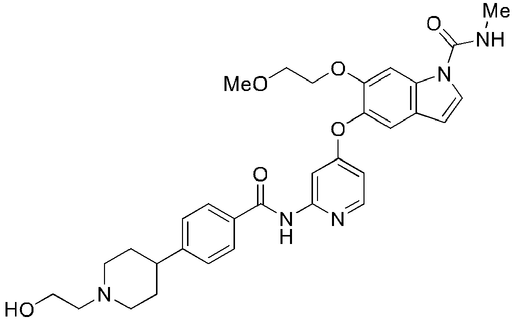

Methanol (268 L) was added to 6-(2-methoxyethoxy)-N-methyl-5-{[2-({[4-(piperidin-4-yl)phenyl]carbonyl}amino)pyridin-4-yl]oxy}-1H-indole-1-carboxamide (18.5 kg, 34.0 mol), and the mixture was stirred at -5°C or lower. Sodium triacetoxyborohydride (21.6 kg, 102 mol, 3.0 eq.) was added in portions under a nitrogen atmosphere, and the mixture was washed in with methanol (9 L). A solution of 1,4-dioxane-2,5-diol (3.3 kg, 27 mol, 0.80 eq.) in methanol (83 L) was added dropwise over a period of 2 hours at an internal temperature of -10 to -5°C, and the mixture was washed in with methanol (9 L) and stirred at an internal temperature of -5 to 0°C for 1 hour. Upon completion of the reaction, water (166.5 kg) was added dropwise to the reaction mixture, and after stirring at 25°C, the mixture was concentrated under reduced pressure to 185 L at an external temperature of 35°C. After adding 1,2-dimethoxyethane (56 L) to the concentrated solution, an aqueous 5 N sodium hydroxide solution (54.4 kg) was added dropwise at an internal temperature of 5°C, and precipitation of crystals was confirmed. After adding n-butanol (370 L) to the suspension and confirming dissolution, the mixture was stirred at an internal temperature of 25°C for 4 hours prior to liquid separation. To the organic layer was added a 10% ethylenediamine aqueous solution (18.5 kg ethylenediamine, 166.5 kg water), and after liquid separation at an internal temperature of 35°C, the mixture was rinsed with water (185.0 kg). To the obtained organic layer were added 1,2-dimethoxyethane (37 L) and water (185.0 kg) prior to liquid separation, and then the organic layer was concentrated under reduced pressure to 105 L at an external temperature of 50°C. Toluene (93 L) was added to the concentrate and the mixture was stirred at an internal temperature of 50°C for 1 hour, after which toluene (278 L) was added dropwise over 1 hour and 12 minutes at an internal temperature of 55°C. After cooling the suspension to an internal temperature of -8°C, it was filtered and rinsed with a mixture of toluene and *n*-butanol (toluene/*n*-butanol = 4.0/1.0 vol., 93 L). The obtained crystals were dried under reduced pressure at 50°C to obtain 16.353 kg of the title compound as a solid.
¹H NMR Spectrum (DMSO-d₆) δ (ppm): 1.60-1.68 (2H, m), 1.71 (2H, br d, J=12.1 Hz), 2.02-2.08 (2H, m), 2.40 (2H, t, J=6.4 Hz), 2.50-2.56 (1H, m), 2.84 (3H, d, J=4.2 Hz), 2.96 (2H, br d, J=11.4 Hz), 3.11 (3H, s), 3.45-3.52 (4H, m), 4.06-4.09 (2H, m), 4.34 (1H, t, J=5.3 Hz), 6.62 (1H, d, J=3.6 Hz), 6.66 (1H, dd, J=5.7, 2.1 Hz), 7.32 (2H, d, J=8.3 Hz), 7.43 (1H, s), 7.68 (1H, d, J=2.3 Hz), 7.77 (1H, d, J=3.8 Hz), 7.89 (2H, d, J=8.3 Hz), 8.07 (1H, s), 8.14 (1H, q, J=4.2 Hz), 8.18 (1H, d, J=5.7 Hz), 10.59 (1H, br s)

### Production Example 5: Production of 5-({2-[({4-[1-(2-hydroxyethyl)piperidin-4-yl]phenyl}carbonyl)amino]pyridin-4-yl}oxy)-6-( 2-methoxyethoxy)-N-methyl-1H-indole-1-carboxamide butanedioate (2:3) (E7090)

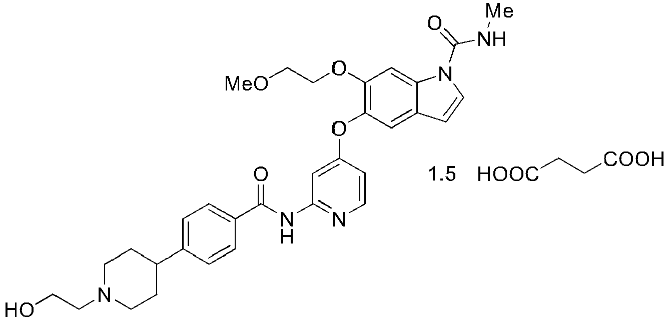

A mixture of 5-({2-[({4-[1-(2-hydroxyethyl)piperidin-4-yl]phenyl}carbonyl)amino]pyridin-4-yl}oxy)-6-( 2-methoxyethoxy)-N-methyl-1H-indole-1-carboxamide (16.353 kg, 27.8 mol) in 2-propanol (45 L) and water (28.1 kg) was stirred at an internal temperature of 45 to 50°C under a nitrogen atmosphere, after which succinic acid (5.9 kg, 50 mol, 1.8 eq.) was added, washed into the mixture with water (1.6 kg), and dissolution was confirmed. The reaction mixture was clarified by filtration and washed in with a mixture of 2-propanol (4 L) and water (6.4 kg), and then 2-propanol (53 L) was added dropwise over 39 minutes at an internal temperature of 35 to 45°C. The mixture was cooled to an internal temperature of 25°C, adding seed crystals (16.4 g) at an internal temperature of 35°C during the cooling. To the suspension was added dropwise 2-propanol (191 L), and the mixture was stirred at an internal temperature of 20°C. The suspension was filtered and rinsed with 2-propanol (128 L). The obtained crystals were dried under reduced pressure at 50°C to obtain 19.032 kg of the title compound (unpulverized) as a solid. The 18.832 kg of the title compound (unpulverized) was pulverized to obtain 18.677 kg of the title compound as a solid.
¹H NMR Spectrum (CDsOD) δ (ppm): 1.97-2.10 (4H, m), 2.53 (6H, s), 2.89-2.97 (4H, m), 2.97-3.05 (2H, m), 3.17 (2H, t, J=5.3 Hz), 3.22 (3H, s), 3.54-3.58 (2H, m), 3.62 (2H, br d, J=12.5 Hz), 3.85-3.89 (2H, m), 4.13-4.16 (2H, m), 6.60 (1H, d, J=3.7 Hz), 6.68 (1H, dd, J=5.9, 2.4 Hz), 7.37 (1H, s), 7.40-7.43 (2H, m), 7.58 (1H, d, J=3.7 Hz), 7.73 (1H, d, J=2.3 Hz), 7.86-7.89 (2H, m), 8.08 (1H, s), 8.14 (1H, d, J=5.8 Hz)

### Test Example 1: Purity test for E7090 (1)

Using a standard sample of E7090 obtained by crystallization (using a particularly high-purity sample of E7090 produced by the method of Production Example 5 as the standard sample) as an external control, the peak areas of each peak for E7090 in the standard sample and in the sample obtained in Production Example 5 were compared to calculate the E7090 content in the sample. In order to calibrate the difference in absorbance per unit mass for each analog, each analog was identified following the procedure described in Test Example 2 and a sample of each analog was synthesized, after which the absorbance (sensitivity coefficient) of each analog was determined against 1 as the absorbance of E7090. In addition, the peak area and sensitivity coefficient values for the analog in the sample were used to calculate the mass% for each analog, and the total for analogs detected above 0.05 mass% was recorded as the total content for all analogs. For analogs without samples, the area% was treated as equivalent to the mass%. The results are shown in Table 1. The retention times and detection limits for each of the analogs in liquid chromatography were as listed in Table 3.

**[Table 1]**

| | Production Example 5 |
|---|---|
| E7090 | 99.9mas% |
| Content of all analogs | 0.12 mass% |

### Liquid chromatography measuring conditions

Detector: Ultraviolet absorptiometer (measuring wavelength: 239 nm).
Column: X-Bridge (Waters Co.), inner diameter: 4.6 mm, length: 25 cm, filler particle size: 5 µm
Column temperature: Constant temperature near 23°C
Mobile phase: Solution A and solution B having the following compositions were eluted with the linear gradient shown in Table 2.
Solution A: Ammonium bicarbonate buffer (pH 10.0)/methanol (1:1, v/v)
Solution B: Methanol
Flow rate: 0.7 mL/min.
Injection rate: 10 µL
Sample rack temperature: Constant temperature near 10°C
Area measurement time: 50 min

**[Table 2]**

| Time (min) | Content ratio of solution B in mobile phase (mPIo) |
|---|---|
| 0 | 26 |
| 30 | 26 |
| 40 | 95 |
| 50 | 95 |
| 50.01 | 26 |
| 70 | STOP |

**[Table 3]**

| Analog | Retertion time (min) | Detection limit (mass%) | Content (mass%) |
|---|---|---|---|
| Compound (2i) | 6.5 | 0.0003 | ≤0.05 |
| Compound (3c) | 13.9 | 0.001 | ≤0.05 |
| Compound(IM-2) | 40.1 | 0.0002 | 0.06 |
| Compound(IM-3) | 7.6 | 0.0002 | ≤0.05 |
| Compound(IM-4) | 41.9 | 0.003 | ≤0.05 |
| Compound(IM-5) | 9.5 | 0.0004 | ≤0.05 |
| Compound (IM-6) | 21.4 | 0.002 | 0.06 |
| Compound (IM-7) | 38 | 0.0004 | ≤0.05 |
| Compound(IM-8) | 46.9 | 0.0004 | ≤0.05 |
| E7090 | 17.3 | 0.0006 | 99.9 |

### Test Example 2: Purity test for E7090 (2)

Purity measurement for E7090 produced as described in PTLs 1 and 2 was carried out under the following conditions. A 10 mg of sample was weighed out into a 10 ml graduated flask, and was precisely adjusted upward in volume with a 50% acetonitrile solution. Separately, 5 ml of the solution was added to a 10 ml graduated flask and precisely adjusted upward in volume. The solutions were analyzed by HPLC. The retention times and area% values obtained by liquid chromatography were as listed in Table 5.

### Liquid chromatography measuring conditions

Detector: Ultraviolet absorptiometer (measuring wavelength: 239 nm).
Column: InertSustain C-18, inner diameter: 4.6 mm, length: 15 cm, filler particle size: 3 µm Column temperature: 40°C
Mobile phase: Solution A and solution B having the following compositions were eluted with the linear gradient shown in Table 4.
Solution A: 12.5 mM phosphate buffer (pH 7)/acetonitrile (9:1, v/v)
Solution B: 12.5 mM phosphate buffer (pH 7)/acetonitrile (1:3, v/v)
Flow rate: 1.0 mL/min.
Injection rate: 10 µL
Sample rack temperature: Constant temperature near 25°C
Area measurement time: 70 min

**[Table 4]**

| Time (min) | Content ratio of solution B in mobile phase (vol%) |
|---|---|
| 0 | 25 |
| 3 | 25 |
| 30 | 45 |
| 50 | 90 |
| 60 | 90 |
| 60.01 | 25 |
| 70 | STOP |

**[Table 5]**

| Retertion time (min) | Area% |
|---|---|
| 10.062 | 0.906 |
| 15251 | 0.113 |
| 15.704 | 0.057 |
| 20.648 | 97.785 |
| 22237 | 0.054 |
| 26.054 | 0287 |
| 31.884 | 0.063 |
| 48.311 | 0.583 |
| 54.165 | 0.151 |
| | 100.000 |

### Test Example 3: Purity test for E7090 (3)

The succinic acid content in E7090 obtained by the method described in Production Example 5 was measured under the following conditions. The results are shown in Table 6. The retention time for succinic acid was 12 minutes based on liquid chromatography.

**[Table 6]**

| | Production Example 5 |
|---|---|
| Succinic acid content | 22 |

### Liquid chromatography measuring conditions

Detector: Ultraviolet absorptiometer (measuring wavelength: 210 nm).
Column: InertSustain AQ-C18 (GL Science), inner diameter: 4.6 mm, length: 25 cm, filler particle size: 5 µm
Column temperature: Constant temperature near 30°C
Mobile phase: Solution A and solution B having the following compositions were eluted with the linear gradient shown in Table 7.
Solution A: Water/phosphoric acid (500:1, v/v)
Solution B: Acetonitrile
Flow rate: 1.0 mL/min.
Injection rate: 10 µL
Sample rack temperature: Constant temperature near 15°C
Area measurement time: 15 min

**[Table 7]**

| Time (min) | Content ratio of solution B in mobile phase (vol%) |
|---|---|
| 0 | 0 |
| 15 | 0 |
| 15.01 | 100 |
| 25 | 100 |
| 25.01 | 0 |
| 40 | STOP |

### Example 2: Analog synthesis

### Production Example 6: Synthesis of 5,5'-{ethane-1,2-diylbis[(piperidine-1,4-diyl)-4,1-phenylenecarbonylazandiylpyridine-2,4-di yloxy]}bis[6-(2-methoxyethoxy)-N-methyl-1H-indole-1-carboxamide](compound (IM-4))

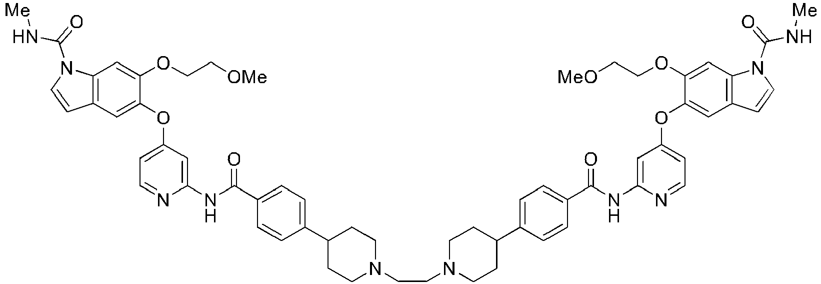

Chloroform (195 mL) was added to 6-(2-methoxyethoxy)-N-methyl-5-{[2-({[4-(piperidin-4-yl)phenyl]carbonyl}amino)pyridin-4-yl]oxy}-1H-indole-1-carboxamide (13.0 g) and the mixture was heated and stirred at 50°C under a nitrogen atmosphere. Glyoxal (2.0 mL) and sodium borohydride (9.50 g) were added, and the mixture was stirred at 50°C for 1 hour. After cooling the reaction mixture to room temperature, it was passed through NH silica gel (205 g) for filtration, and the NH silica gel was rinsed with chloroform (1300 mL). The filtrate was concentrated under reduced pressure at 40°C, and then tetrahydrofuran (65 mL) and methanol (65 mL) were added to the concentrated residue and the resulting suspension was stirred at 60°C. After cooling to room temperature, the suspension was filtered and rinsed by addition of a mixture of tetrahydrofuran and methanol (tetrahydrofuran/methanol = 1/1, 39 mL). The obtained solid was dried under reduced pressure to obtain 8.14 g of the title compound (61.1%).
¹H NMR (DMSO-d₆) δ (ppm): 1.60-1.68 (4H, m), 1.73 (4H, br d, J=11.7 Hz), 2.00-2.07 (4H, m), 2.45 (4H, s), 2.51-2.57 (2H, m), 2.84 (6H, d, J=4.5 Hz), 2.98 (4H, br d, J=11.0 Hz), 3.12 (6H, s), 3.45-3.49 (4H, m), 4.05 -4.09 (4H, m), 6.62 (2H, d, J=3.8 Hz), 6.66 (2H, dd, J=5.7, 2.3 Hz), 7.33 (4H, d, J=8.3 Hz), 7.43 (2H, s), 7.68 (2H, d, J=2.3 Hz), 7.77 (2H, d, J=3.4 Hz), 7.89 (4H, d, J=7.9 Hz), 8.07 (2H, s), 8.14 (2H, q, J=4.5 Hz), 8.19 (2H, d, J=6.0 Hz), 10.60 (2H, br s)

### Production Example 7: Synthesis of 4-[2-(4-f4-[(4-f[6-(2-methoxyethoxy)-1-(methylcarbamoyl)-1H-indol-5-yl]oxylpyridin-2-yl )carbamoyl]phenyl}piperidin-1-yl)ethoxy1-4-oxobutanoic acid (compound (IM-5))

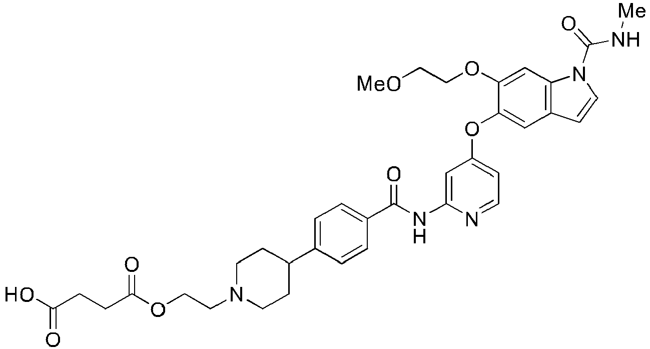

After adding tetrahydrofuran (20 mL), triethylamine (2.9 mL) and N,N-dimethylaminopyridine (83 mg) to 5-({2-[({4-[1-(2-hydroxyethyl)piperidin-4-yl]phenyl}carbonyl)amino]pyridin-4-yl}oxy)-6-( 2-methoxyethoxy)-N-methyl-1H-indole-1-carboxamide (4.01 g) and succinic anhydride (1.23 g) under a nitrogen atmosphere, the mixture was stirred at 60°C for 3 hours. The reaction mixture was cooled on ice bath and the precipitated solid was filtered and rinsed with tetrahydrofuran (8 mL). The obtained solid was dried under reduced pressure to obtain 4.76 g of the title compound.
¹H NMR (DMSO-d₆) δ (ppm): 1.60-1.77 (4H, m), 2.09-2.15 (2H, m), 2.45-2.56 (5H, m), 2.58 (2H, t, J=6.0 Hz), 2.84 (3H, d, J=4.5 Hz), 2.97 (2H, br d, J=11.0 Hz), 3.11 (3H, s), 3.45-3.49 (2H, m), 4.05-4.09 (2H, m), 4.13 (2H, t, J=6.1 Hz), 6.62 (1H, d, J=3.4 Hz), 6.66 (1H, dd, J=5.7, 2.4 Hz), 7.33 (2H, d, J=8.3 Hz), 7.43 (1H, s), 7.68 (1H, d, J=2.3 Hz), 7.77 (1H, d, J=3.4 Hz), 7.89 (2H, d, J=8.3 Hz), 8.07 (1H, s), 8.15 (1H, q, J=4.2 Hz), 8.18 (1H, d, J=5.7 Hz), 10.61 (1H, br s)

### Production Example 8: Synthesis of 4-[1-(2-hydroxyethyl)piperidin-4-yl]-N-(4-([6-(2-methoxyethoxy)-1H-indol-5-yl]oxylpyridi n-2-yl)benzamide (compound (IM-6))

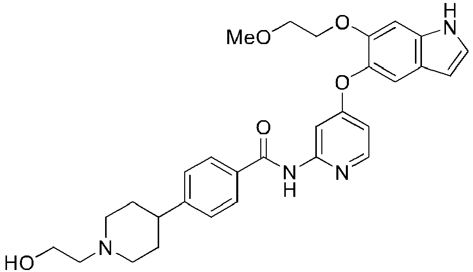

Tetrahydrofuran (100 mL) and tetrabutylammonium fluoride (128 mL, 1 mol/L, 128 mmol, 5.0 eq.) were added to 5-({2-[({4-[1-(2-hydroxyethyl)piperidin-4-yl]phenyl}carbonyl)amino]pyridin-4-yl}oxy)-6-( 2-methoxyethoxy)-N-methyl-1H-indole-1-carboxamide (15.0 g, 25.5 mmol), and the mixture was stirred at 50°C. After cooling to room temperature, ethyl acetate (300 mL) and water (100 mL) were added, prior to liquid separation. The organic layer was rinsed 7 times with aqueous 5% sodium hydrogencarbonate (150 mL) and water (150 mL). The organic layer was concentrated under reduced pressure and the obtained residue was purified by NH-silica gel chromatography (NH-silica gel, ethyl acetate:methanol = 20:1 → 10:1 → 1:1). The target fraction was concentrated under reduced pressure to obtain 1.81 g of the title compound as a solid (13.4%).
¹H NMR (DMSO-d₆) δ (ppm): 1.60-1.68 (2H, m), 1.72 (2H, br d, J=11.7 Hz), 2.01-2.09 (2H, m), 2.40 (2H, t, J=6.4 Hz), 2.51-2.57 (1H, m), 2.96 (2H, br d, J=11.0 Hz), 3.12 (3H, s), 3.43-3.53 (4H, m), 4.03-4.08 (2H, m), 4.34 (1H, t, J=5.3 Hz), 6.35-6.38 (1H, m), 6.62 (1H, dd, J=5.7, 2.3 Hz), 7.14 (1H, s), 7.28 (1H, dd, J=2.7, 2.3 Hz), 7.32 (2H, d, J=8.3 Hz), 7.34 (1H, s), 7.67 (1H, d, J=2.3 Hz), 7.88 (2H, d, J=7.9 Hz), 8.16 (1H, d, J=5.7 Hz), 10.56 (1H, br s), 11.05 (1H, br s)

### Production Example 9: Synthesis of 5-({2-[4-(1-ethylpiperidin-4-yl)benzamide]pyridin-4-yl}oxy)-6-(2-methoxyethoxy)-N-meth yl-1H-indole-1-carboxamide (compound (IM-7))

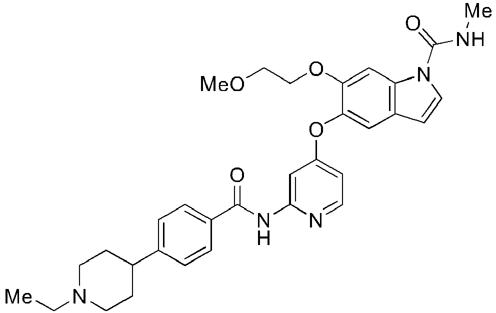

Methanol (300 mL) was added to 6-(2-methoxyethoxy)-N-methyl-5-{[2-({[4-(piperidin-4-yl)phenyl]carbonyl}amino)pyridin-4-yl]oxy}-1H-indole-1-carboxamide (15.00 g, 27.59 mmol), and the mixture was stirred at 0°C. Acetaldehyde (4.70 mL, 8.38 mmol, 3.04 eq.) and sodium triacetoxyborohydride (17.55 g, 82.81 mol, 3.00 eq.) were added and the mixture was stirred for 4 hours. Water (135 mL) was added to the reaction mixture, which was then stirred at room temperature and subsequently concentrated under reduced pressure to about 135 mL at 35°C. Tetrahydrofuran (210 mL) and toluene (105 mL) were added to the concentrated solution, and then an aqueous 5 N sodium hydroxide solution (52.5 mL) was added at 0°C prior to liquid separation. After rinsing the organic layer two times with water (150 mL), it was concentrated under reduced pressure at 35°C. The obtained residue was purified by NH-silica gel chromatography (682 g NH-silica gel, ethyl acetate:methanol = 100:0 → 100:1 → 29:1). The target fraction was concentrated under reduced pressure at 35°C. Tetrahydrofuran (28.9 mL) was added to the obtained concentrated residue, and after stirring to dissolution at 60°C, the mixture was cooled on ice bath, methyl *tert*-butyl ether (57.8 mL) was added, and stirring was continued. The suspension was filtered and the filtered solid was rinsed with a mixture of tetrahydrofuran (4.8 mL) and methyl *tert*-butyl ether (9.6 mL). The obtained solid was dried under reduced pressure at 50°C to obtain 8.82 g of the title compound (56.0%).
¹H NMR (DMSO-d₆) δ (ppm): 1.00 (3H, t, J=7.2 Hz), 1.64 (2H, qd, J=12.3, 4.4 Hz), 1.71-1.77 (2H, m), 1.94 (2H, td, J=11.5, 1.9 Hz), 2.34 (2H, q, J=7.2 Hz), 2.51-2.56 (1H, m), 2.84 (3H, d, J=4.5 Hz), 2.96 (2H, br d, J=11.5 Hz), 3.12 (3H, s), 3.45-3.49 (2H, m), 4.05-4.09 (2H, m), 6.62 (1H, d, J=3.6 Hz), 6.66 (1H, dd, J=5.7, 2.3 Hz), 7.33 (2H, d, J=8.3 Hz), 7.43 (1H, s), 7.68 (1H, d, J=2.3 Hz), 7.77 (1H, d, J=3.6 Hz), 7.89 (2H, d, J=8.3 Hz), 8.07 (1H, s), 8.14 (1H, q, J=4.2 Hz), 8.18 (1H, d, J=5.7 Hz), 10.60 (1H, br s)

### Production Example 10: Synthesis of 5,5'-{(2-methylpropane-1,3-diyl)bis[(piperidine-1,4-diyl)-4,1-phenylenecarbonylazanediylpy ridine-2,4-diyloxy]}bis[6-(2-methoxyethoxy)-N-methyl-1H-indole-1-carboxamide] (compound (IM-8))

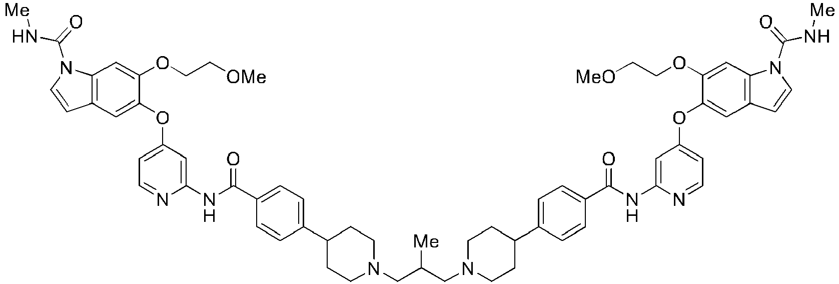

Tetrahydrofuran (30 mL), diazabicycloundecene (0.17 mL, 1.1 mmol, 0.2 eq.) and methacrolein (0.69 mL, 8.3 mmol, 1.5 eq.) were added to 6-(2-methoxyethoxy)-N-methyl-5-{[2-({[4-(piperidin-4-yl)phenyl]carbonyl}amino)pyridin-4-yl]oxy}-1H-indole-1-carboxamide (3.0 g, 5.5 mmol) and the reaction mixture was stirred at room temperature for 16 hours and then concentrated under reduced pressure at 40°C. To the concentrated residue were added 6-(2-methoxyethoxy)-N-methyl-5-{[2-({[4-(piperidin-4-yl)phenyl]carbonyl}amino)pyridin-4-yl]oxy}-1H-indole-1-carboxamide (2.4 g, 4.4 mmol, 0.8 eq.) and methanol (34 mL), and the mixture was ice-cooled. Sodium triacetoxyborohydride (3.51 g, 16.6 mmol, 3.0 eq.) was added under a nitrogen atmosphere, and the mixture was stirred for 2.5 hours while cooling on ice bath. Water (20 mL) was added to the reaction mixture, which was then concentrated under reduced pressure to 40°C. Tetrahydrofuran (50 mL) was added to the concentrated solution, and then an aqueous 5 N sodium hydroxide solution (25 mL) was added at 0°C prior to liquid separation. After rinsing the organic layer two times with brine, it was concentrated under reduced pressure at 40°C. The obtained residue was dissolved in tetrahydrofuran and purified by NH-silica gel chromatography (110 g NH-silica gel, ethyl acetate:methanol = 100:0 → 95:5). The target fraction was concentrated under reduced pressure at 40°C. Methanol (200 mL) was added to the obtained concentrated residue, and the resulting suspension was stirred at room temperature. The suspension was filtered, and the filtered solid was rinsed with methanol and then dried under reduced pressure at 40°C to obtain 2.19 g of the title compound (39.7%).
¹H NMR (DMSO-d₆) δ (ppm): 0.89 (3H, d, J=6.2 Hz), 1.59-1.69 (4H, m), 1.73 (4H, br d, J=11.0 Hz), 1.87-1.96 (3H, m), 1.98-2.07(4 H, m), 2.26 (2H, dd, J=11.9, 5.7 Hz), 2.50-2.57 (2H, m), 2.84 (6H, d, J=4.4 Hz), 2.88-2.95 (4H, m), 3.11 (6H, s), 3.45-3.49 (4H, m), 4.05-4.09 (4H, m), 6.62 (2H, d, J=3.5 Hz), 6.66 (2H, dd, J=5.7, 2.2 Hz), 7.33 (4H, d, J=8.4 Hz), 7.44 (2H, s), 7.68 (2H, d, J=2.2 Hz), 7.77 (2H, d, J=4.0 Hz), 7.89 (4H, d, J=8.4 Hz), 8.07 (2H, s), 8.16 (2H, q, J=4.4 Hz), 8.18 (2H, d, J=5.7 Hz), 10.63 (2H, br s)

### Production Example 11: Synthesis of 5-({2-[4-(1-tert-butylpiperidin-4-yl)benzarnide]pyridin-4-yl}oxy)-6-(2-methoxyethoxy)-N-methyl-1H-indole-1-carboxamide (compound (IM-2))

### Production Example 11-1: Synthesis of tert-butyl 4-{4[(benzyloxy)carbonyl]phenyl}piperidine-1-carboxylate compound (21-1))

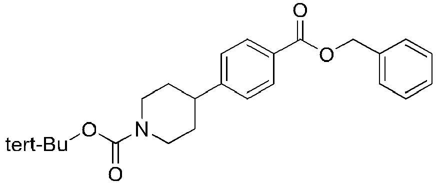

After adding N,N-dimethylformamide (1150 mL), potassium carbonate (67.7 g, 489.6 mmol, 1.30 eq.) and benzyl bromide (49.2 mL, 414.2 mmol, 1.10 eq.) to 4-(1-(*tert*-butoxycarbonyl)piperidin-4-yl)benzoic acid (115.0 g, 376.6 mmol), the mixture was stirred at room temperature for 21 hours. Ethyl acetate (4.6 L) and water (2.6 L) were added to the reaction mixture prior to liquid separation. The organic layer was rinsed with water (690 mL) and then rinsed with brine (690 mL water, 13.8 g sodium chloride). The mixture was concentrated under reduced pressure at 40°C, ethyl acetate (230 mL) was added to the concentrated residue, and the mixture was stirred to dissolution at 70°C, after which it was cooled to room temperature and stirring was continued. After cooling to 0°C, n-heptane (920 mL) was added dropwise. The suspension was filtered and the filtered solid was rinsed with a mixture of ethyl acetate and n-heptane (ethyl acetate/n-heptane = 1/4, 230 mL). The obtained solid was dried under reduced pressure at 40°C to obtain 120.4 g (80.8%) of the title compound.

### Production Example 11-2: Synthesis of benzyl 4-[1-(2-cyanopropan-2-yl)piperidin-4-yl]benzoate (compound (21-2))

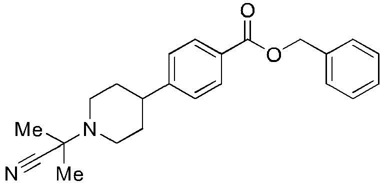

After adding *tert*-butyl 4-{4[(benzyloxy)carbonyl]phenyl}piperidine-1-carboxylate (compound (21-1)) (120.4 g, 304.4 mmol) to trifluoroacetic acid (241 mL) while cooling on ice bath under a nitrogen atmosphere and stirring for 1 hour, the reaction mixture was concentrated under reduced pressure at 35°C. Water (310 mL), toluene (602 mL) and tetrahydrofuran (602 mL) were added to the concentrated solution, and then a 25% aqueous sodium hydroxide solution (146 mL) was added while cooling on ice bath, prior to liquid separation. After re-extraction from the aqueous layer with a mixture of tetrahydrofuran and toluene (tetrahydrofuran/toluene = 1/1, 602 mL), and the combined organic layer was washed with water (240 mL) and concentrated under reduced pressure at 40°C to obtain an oil (90.5 g).
After adding acetone (680 mL) and acetone cyanohydrin (27.2 mL, 297.9 mol) to the obtained oil (80.0 g) and stirring at room temperature for 2 days, the reaction mixture was concentrated under reduced pressure at 40°C. Methanol (240 mL) was added to the concentrated residue and the mixture was stirred at 0°C. The suspension was filtered and the filtered solid was rinsed with methanol (160 mL). The obtained solid was dried under reduced pressure at 40°C to obtain 90.5 g (93.4%) of the title compound.

### Production Example 11-3: Synthesis of benzyl 4-(1-tert-butyl)piperidin-4-yl)benzoate (compound (21-3))

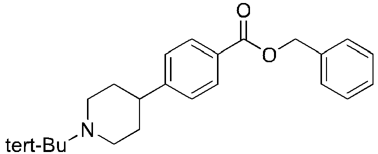

Toluene (23 g) and tetrahydrofuran (900 mL) were added to compound (21-2) (90.0 g, 248.3 mmol), and the mixture was stirred at -40°C. A solution of methylmagnesium bromide in tetrahydrofuran (730 mL, 1.06 mol/L, 774.1 mmol, 3.1 eq.) was added dropwise under a nitrogen atmosphere, and the mixture was stirred at -25°C for 9 hours. The reaction mixture was added to a mixture of toluene (1630 mL) and aqueous ammonium acetate (163 g ammonium acetate, 815 mL water) while cooling on ice bath and stirred, prior to liquid separation. After rinsing the organic layer with water (270 mL), it was concentrated under reduced pressure at 40°C. Methanol (250 mL) was added to the concentrated residue and the mixture was stirred to dissolution at 60°C, after which it was cooled on ice bath and further stirred. The suspension was filtered and the filtered solid was rinsed with methanol (50 mL). The obtained filtrate was concentrated under reduced pressure at 40°C and then purified by NH-silica gel chromatography (600 g NH-silica gel, ethyl acetate: n-heptane = 1:9). The target fraction was concentrated under reduced pressure at 40°C. Methanol (50 mL) was added to the obtained concentrated residue and the mixture was stirred to dissolution at 60°C, after which it was cooled on ice bath and further stirred. The suspension was filtered and the filtered solid was rinsed with methanol (30 mL). The obtained filtrate was concentrated under reduced pressure at 40°C to obtain 19.1 g (21.9%) of the title compound as a solid.

### Production Example 11-4: Synthesis of benzyl 4-(1-tert-butyl)piperidin-4-yl)benzoate (compound (214))

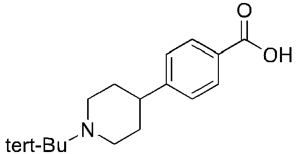

Methanol (348 mL) and water (35 mL) were added to compound (21-3) (19.1 g, 54.3 mmol), and the mixture was stirred. After adding 10% palladium carbon (2.87 g, 15 wt%), the mixture was further stirred at room temperature for 16.6 hours under a hydrogen atmosphere. Water (99 mL) was added to the reaction mixture, which was then filtered with Celite and rinsed with a mixture of methanol and water (methanol/water = 2/1, 300 mL). The obtained filtrate was concentrated under reduced pressure at 40°C. The concentrated residue was subjected to azeotropic distillation twice with toluene (100 mL) and methanol (50 mL), twice with toluene (50 mL) and methanol (10 mL) and once with toluene (50 mL), at 40°C. Methanol (57 mL) was added to the obtained concentrated residue and the suspension was stirred at 70°C, after which it was cooled to 0°C and further stirred. The suspension was filtered and the filtered solid was rinsed with methanol (5 mL). The obtained solid was dried under reduced pressure at 40°C to obtain 0.87 g (6.2%) of the title compound. The obtained filtrate was concentrated under reduced pressure at 40°C, and then tetrahydrofuran (73 mL) was added, and the mixture was stirred at 50°C, cooled to room temperature and further stirred. The suspension was filtered, and the filtered solid was rinsed with tetrahydrofuran (20 mL) and then dried under reduced pressure at 40°C to obtain 4.22 g (29.7%) of the title compound.

### Production Example 11-5: Synthesis of 5-({2-[4-(1-tert-butylpiperidin-4-yl)benzamide]pyridin-4-yl}oxy)-6-(2-methoxyethoxy)-N-methyl-1H-indole-1-carboxamide (compound (IM-2))

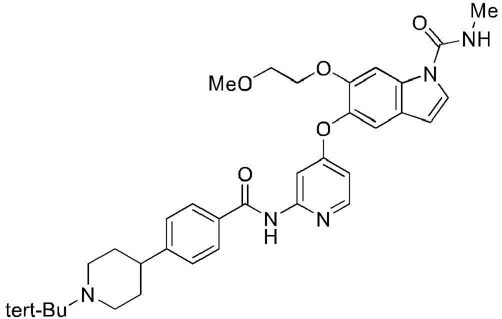

Triethylamine (4.3 mL, 31 mmol, 2.0 eq.) was added to a suspension of 5-((2-aminopyridin-4-yl)oxy)-6-(2-methoxyethoxy)-N-methyl-1H-indole-1-carboxamide methanesulfonate (7.30 g, 15.4 mmol), compound (21-3) (4.84 g, 18.5 mmol, 1.2 eq.), N,N-dimethyl-4-aminopyridine (3.77 g, 31 mmol, 2.0 eq.) and 1-ethyl-3-(3-dimethylaminopropyl)carbodiimide hydrochloride (5.92 g, 31.0 mmol, 2.0 eq.) in 1,2-dimethoxyethane (55 mL) under a nitrogen atmosphere, and the mixture was stirred at 60°C for 22 hours. The reaction mixture was cooled to room temperature, tetrahydrofuran (110 mL) and toluene (55 mL) were added, and then an aqueous 2 N sodium hydroxide solution (33.3 g) was added prior to liquid separation. After rinsing the obtained organic layer 3 times with water (30 mL), it was concentrated under reduced pressure at 40°C. The obtained residue was dissolved in tetrahydrofuran and purified by NH-silica gel chromatography (500 g NH-silica gel, ethyl acetate:n-heptane = 6:1). The target fraction was concentrated under reduced pressure at 40°C. Ethanol (65 mL) was added to the obtained concentrated residue and the suspension was stirred at 40°C, after which it was stirred while cooling on ice bath. The suspension was filtered and the filtered solid was rinsed with ethanol (22 mL). The obtained solid was dried under reduced pressure at 40°C to obtain 6.50 g (67.2%) of the title compound.
¹H NMR (DMSO-d₆) δ (ppm): 1.02 (9H, s), 1.58 (2H, qd, J=12.2, 3.1 Hz), 1.76 (2H, br d, J=12.8 Hz), 2.11 (2H, dd, J=11.0, 11.0 Hz), 2.48-2.52 (1H, m), 2.84 (3H, d, J=4.4 Hz), 3.08 (2H, br d, J=11.0 Hz), 3.11 (3H, s), 3.45-3.49 (2H, m), 4.05-4.09 (2H, m), 6.62 (1H, d, J=3.5 Hz), 6.66 (1H, dd, J=5.7, 2.6 Hz), 7.32 (2H, d, J=8.4 Hz), 7.44 (1H, s), 7.68 (1H, d, J=2.2 Hz), 7.77 (1H, d, J=3.5 Hz), 7.88 (2H, d, J=8.4 Hz), 8.07 (1H, s), 8.15 (1H, q, J=4.0 Hz), 8.18 (1H, d, J=5.7 Hz), 10.62 (1H, br s)

### Example 3: Synthesis of 4-(1-tert-butoxycarbonyl)piperidin-4-yl)benzoic acid (compound (3a-1))

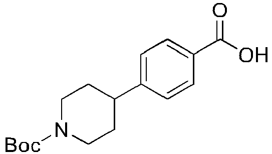

A microreactor apparatus was used as shown in Fig. 1 to carry out flow reaction to obtain 1-N-(*tert-*butoxycarbonyl)-4-(4'-carboxyphenyl)piperidine from N-(*tert*-butoxycarbonyl)-4-(4-bromophenyl)piperidine. A solution of *N*-(*tert*-butoxycarbonyl)-4-(4-bromophenyl)piperidine (0.75 g, 2.2 mmol) in dehydrated THF (45 ml) was adjusted to a 0.05 mol concentration (solution A). A 2.64 mol concentration solution of *n*-BuLi in *n*-hexane (2 ml) was diluted with dehydrated hexane (10 ml) to a 0.44 mol concentration (solution B). The methanol used was an HPLC-grade product. Mixer 1 was a DH mixer by Nakamura Choukou Co., Ltd., mixer 2 was a Y-mixer with an inner diameter of 1 mmϕ, and mixer 3 was a T-mixer with an inner diameter of 1 mmϕ. Carbon dioxide gas was supplied with a massflow controller (CR-100 FLOW COMPO by Kofloc Kyoto), liquid delivery was with a syringe pump (KDS-200 by KD Scientific Inc.) and a gas-tight syringe (SGE syringe by Trajan), and all of the piping used was PFA with an inner diameter of 1.0 mm and an outer diameter of 1/16-inch. Methanol was delivered using a plunger pump (UI-22 by Tokyo Rikakikai Co., Ltd.). The piping length connecting mixer 1 with mixer 2 and mixer 2 with mixer 3 was 23 cm, and the piping length from mixer 3 to the outlet was 4 cm. Mixer 1, mixer 2 and mixer 3 were embedded in a thermobath at -41°C, and the piping for solution A to be introduced into mixer 1 was embedded to about 50 cm while the piping for solution B was embedded to about 20 cm, and pre-cooled. Solution A was delivered at a flow rate of 2 ml/min while solution B was delivered at a flow rate of 0.329 ml/min (1.5 eq), and the two were mixed by mixer 1 at -41°C. The carbon dioxide gas was introduced into mixer 2 at a flow rate of 16 ml/min (7.3 eq) and reacted with the mixture of solution A and solution B. Methanol was introduced into mixer 3 at a flow rate of 0.5 ml/min and combined with the reaction mixture to suspend the reaction. Based on calibration curve HPLC quantitative analysis for the solution obtained by flow reaction for about 17 minutes, the title compound was obtained in 95.5% yield.

### Formulation Example

E7090 produced by the production process of the invention was formulated into formulations 1 to 5 listed in Table 8 below, based on a known method such as the method described in General Rules for Preparations according to the Japanese Pharmacopoeia, 18th Edition. The units in the table are mg.

**Table 8**

| Component | Formulation 1 | Formulation 2 | Formulation 3 | Formulation 4 | Formulation 5 |
|---|---|---|---|---|---|
| E7090 | 35.0 | 35.0 | 35.0 | 35.0 | 35.0 |
| Lactose hydrate | 67.8 | 122.8 | 112.7 | 144.3 | 145.7 |
| Polyvinyl alcohol (partially saponified) | 1.88 | 2.40 | 4.00 | 2.50 | 2.80 |
| Low-substituted hydroxypropyl cellulose | 19.3 | 30.0 | 20.0 | 22.0 | 12.0 |
| Macogol4000 | 0.949 | 1212 | 2.020 | 1.616 | 1.454 |
| Hypromellose | 5.1 | 10.0 | 12.0 | 32 | 2.9 |
| Talc | 0.696 | 0.888 | 1.480 | 1.184 | 1.066 |
| Titanium oxide | 1.080 | 1.378 | 2297 | 1.838 | 1.654 |
| Magnesium stearate | 1.1 | 22 | 1.0 | 12 | 12 |
| Yellow iron sesquioxide | 0.095 | 0.122 | 0203 | 0.162 | 0.146 |
| Total | 133.0 | 206.0 | 190.7 | 213.0 | 203.9 |

## Claims

1. A process for producing compound (3d): 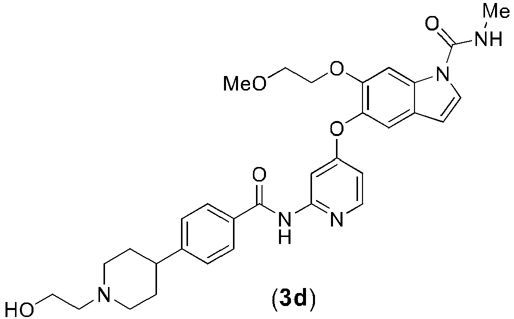 or a salt thereof, wherein the process comprises:
a) step a) in which compound (2i): 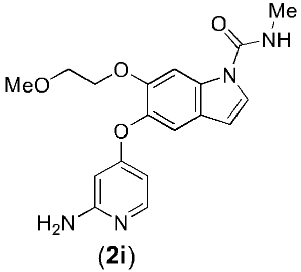 or a salt thereof and compound (3a): 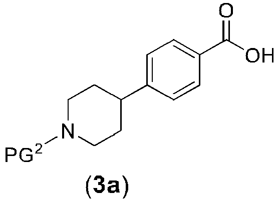 (where PG² represents a nitrogen protecting group)
are reacted in the presence of a condensation agent, to produce compound (3b): 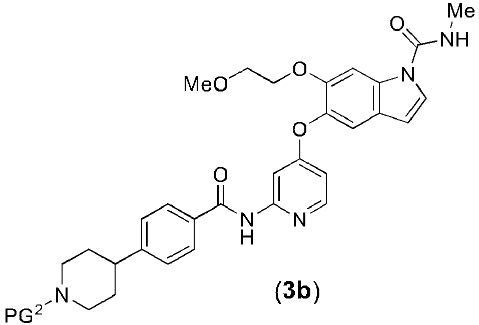 (where PG² represents the same group as specified above),
b) step b) in which PG² in compound (3b) obtained in step a) is removed to produce compound (3c): 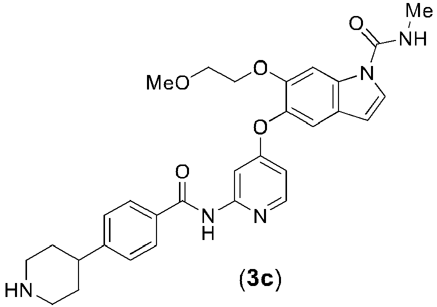
c) step c) in which compound (3c) obtained in step b) and a hydroxyethylating agent are reacted to produce compound (3d): 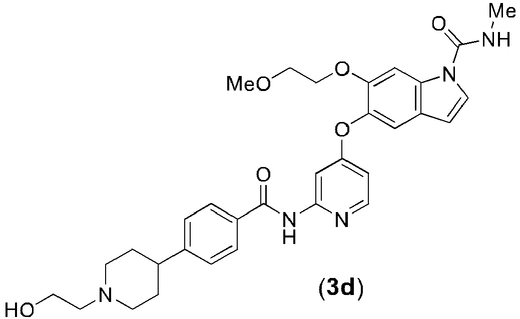 and
d) if necessary, step d) in which compound (3d) obtained in step c) is converted to a pharmaceutically acceptable salt.

2. The production process according to claim 1, wherein PG² is a tert-butoxycarbonyl group.

3. The production process according to claim 1, wherein the condensation agent is 1-ethyl-3-(3-dimethylaminopropyl)carbodiimide hydrochloride.

4. The production process according to claim 2, wherein formic acid or hydrochloric acid is used in step b).

5. The production process according to claim 1, wherein the hydroxyethylating agent is 1,4-dioxane-2,5-diol, and step c) further includes the use of a reducing agent.

6. The production process according to claim 1, wherein the pharmaceutically acceptable salt is a succinate.

7. Compound (3d) or a salt thereof, wherein the content of compound (IM-7) is 0.48 mass% or lower.

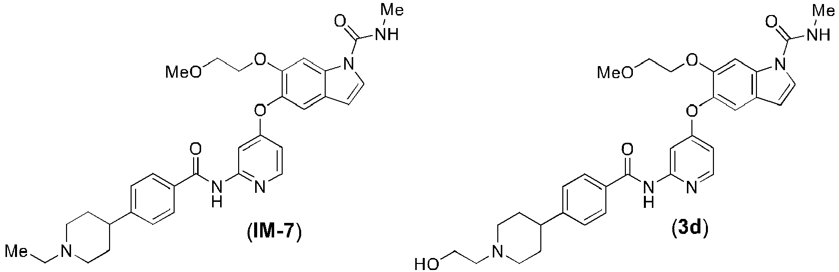

8. Compound (3d) or a salt thereof, wherein the content of compound (IM-5) is 0.40 mass% or lower.

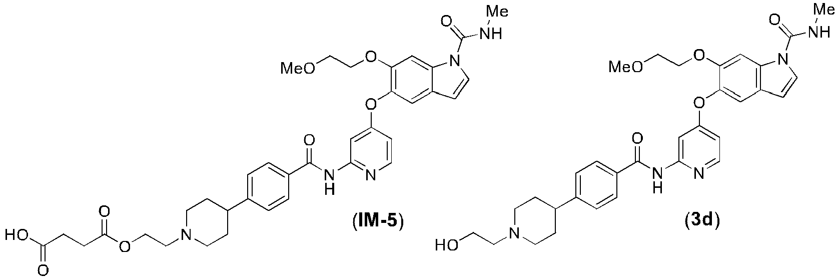

9. Compound (3d) or a salt thereof, wherein the content of compound (IM-2) is 0.30 mass% or lower.

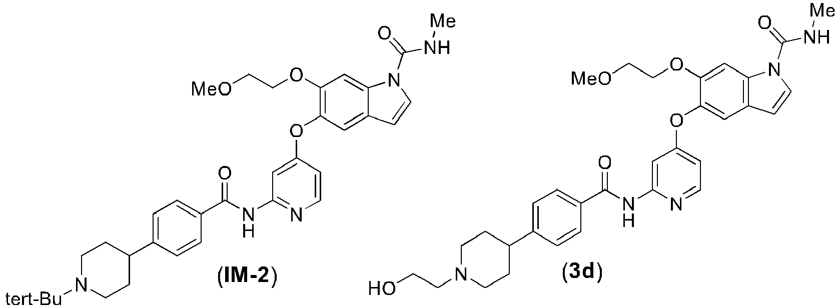

10. Compound (3d) or a salt thereof, wherein the content of compound (3c) is 0.30 mass% or lower.

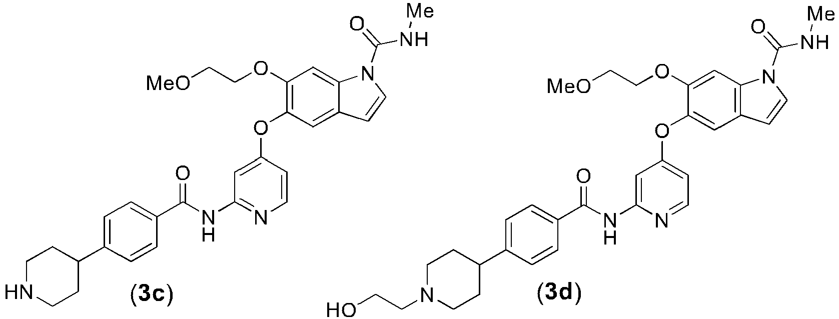

11. Compound (3d) or a salt thereof, wherein the content of compound (IM-3) is 0.30 mass% or lower.

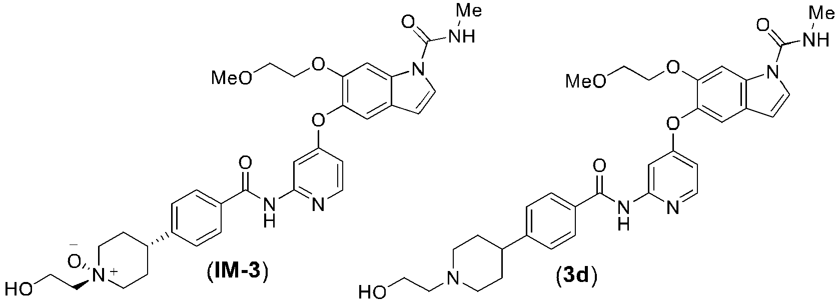

12. Compound (3d) or a salt thereof, wherein the content of all analogs is 2.0 mass% or lower.

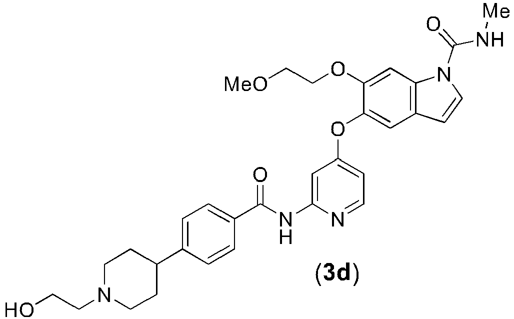

13. Compound (3d) or a salt thereof, wherein the content of compound (IM-7) is 0.48 mass% or lower and the content of all analogs is 2.0 mass% or lower.

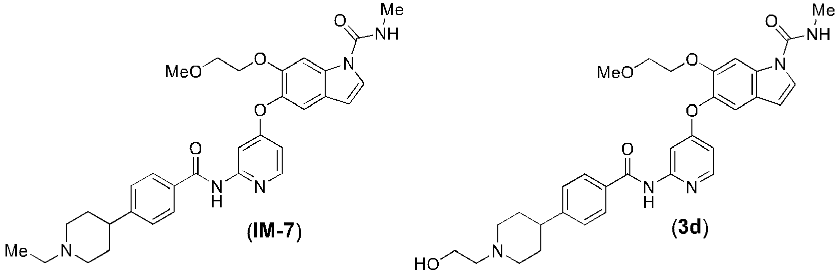

14. Compound (3d) or a salt thereof, wherein the content of compound (IM-5) is 0.40 mass% or lower and the content of all analogs is 2.0 mass% or lower.

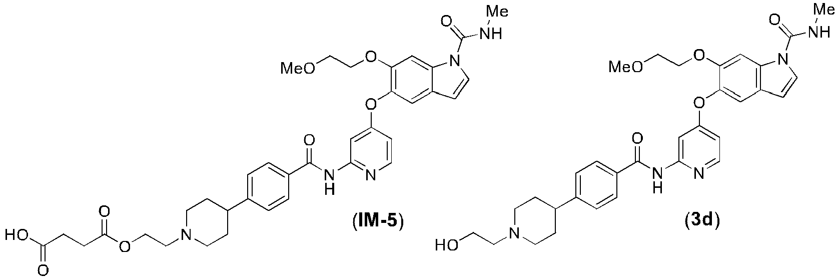

15. Compound (3d) or a salt thereof, wherein the content of compound (IM-2) is 0.30 mass% or lower and the content of all analogs is 2.0 mass% or lower.

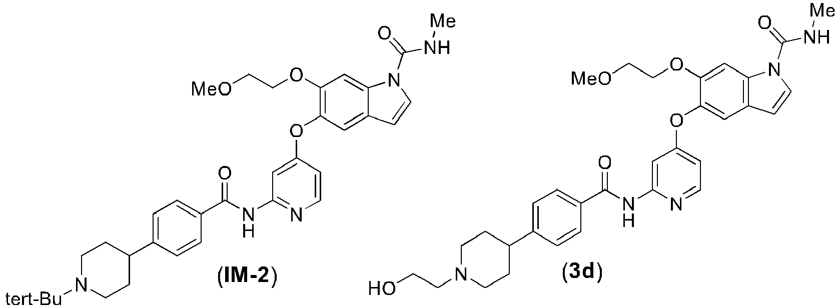

16. Compound (3d) or a salt thereof, wherein the content of compound (3c) is 0.30 mass% or lower and the content of all analogs is 2.0 mass% or lower.

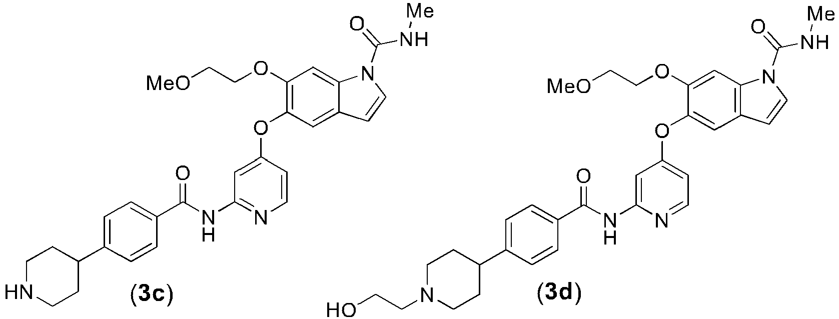

17. Compound (3d) or a salt thereof, wherein the content of compound (IM-3) is 0.30 mass% or lower and the content of all analogs is 2.0 mass% or lower.

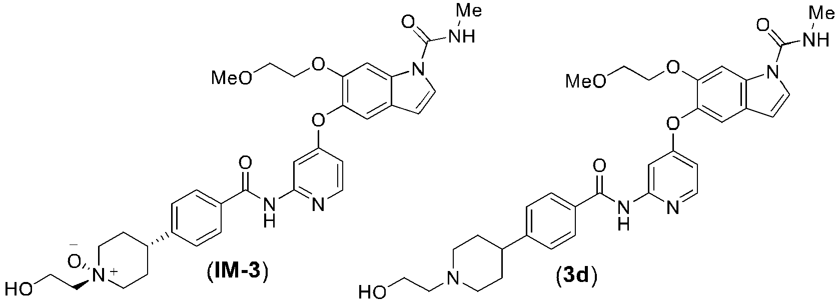

18. Compound (3d) or a salt thereof, wherein the content of compound (3d) or a salt thereof is 97.0 mass% or greater.

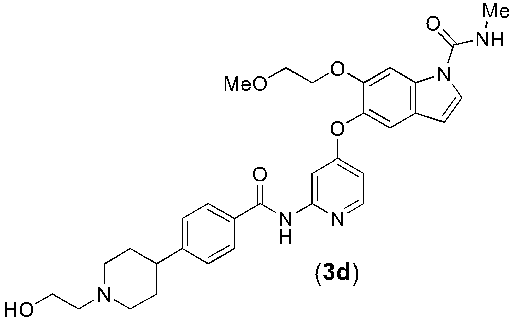

19. Compound (3d) or a salt thereof, wherein the content of compound (3d) or a salt thereof is 97.0 mass% or greater and the content of compound (IM-7) is 0.48 mass% or lower.

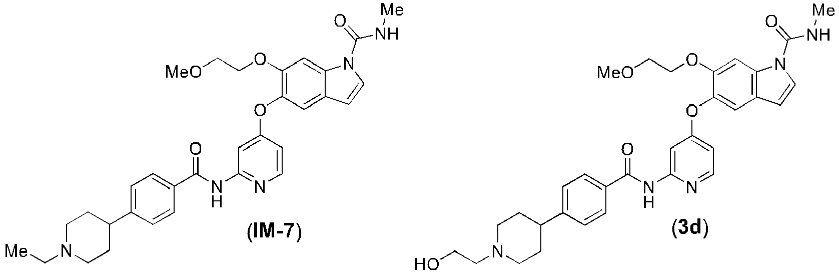

20. Compound (3d) or a salt thereof, wherein the content of compound (3d) or a salt thereof is 97.0 mass% or greater and the content of compound (IM-5) is 0.40 mass% or lower.

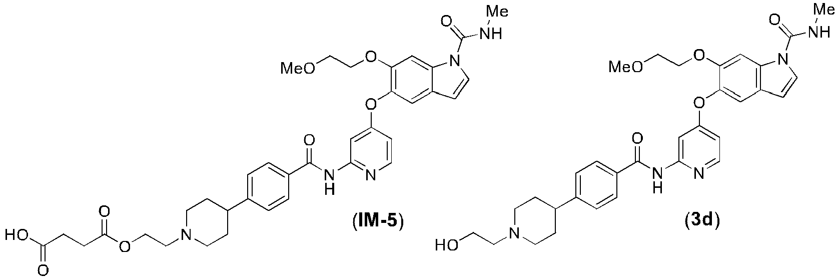

21. Compound (3d) or a salt thereof, wherein the content of compound (3d) or a salt thereof is 97.0 mass% or greater and the content of compound (IM-2) is 0.30 mass% or lower.

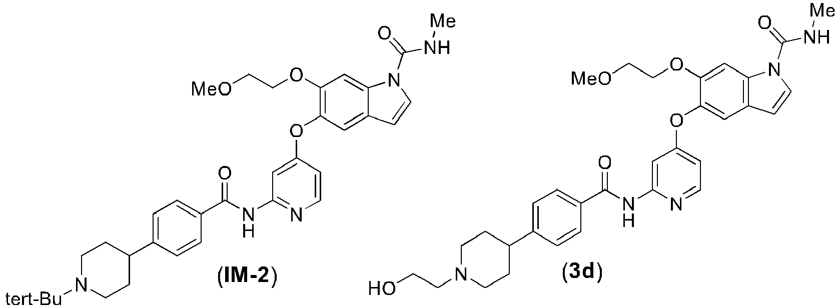

22. Compound (3d) or a salt thereof, wherein the content of compound (3d) or a salt thereof is 97.0 mass% or greater and the content of compound (3c) is 0.30 mass% or lower.

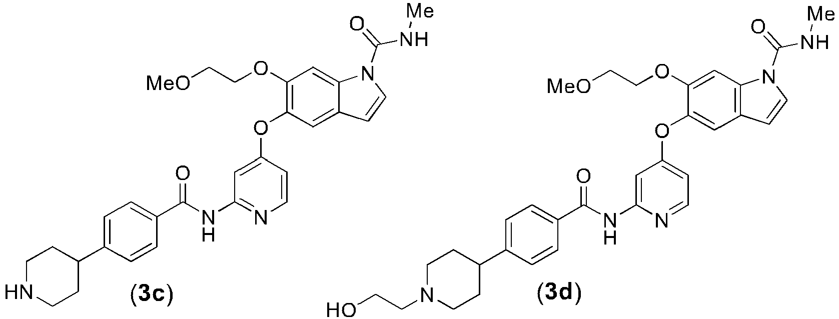

23. Compound (3d) or a salt thereof, wherein the content of compound (3d) or a salt thereof is 97.0 mass% or greater and the content of compound (IM-3) is 0.30 mass% or lower.

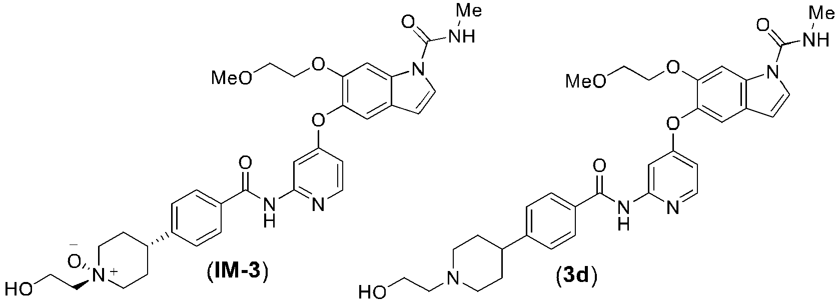

24. Compound (3d) or a salt thereof, wherein the content of compound (3d) or a salt thereof is 97.0 mass% or greater, the content of compound (IM-7) is 0.48 mass% or lower, and the content of all analogs is 2.0 mass% or lower.

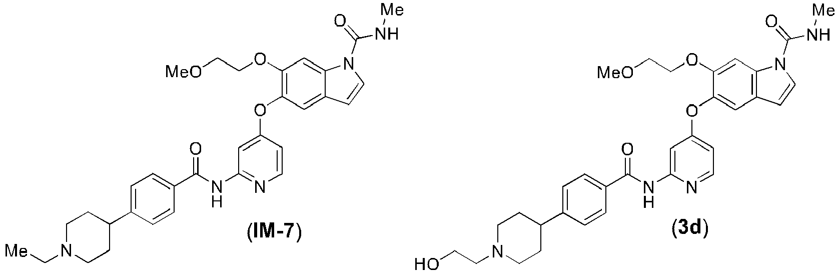

25. Compound (3d) or a salt thereof, wherein the content of compound (3d) or a salt thereof is 97.0 mass% or greater, the content of compound (IM-5) is 0.40 mass% or lower, and the content of all analogs is 2.0 mass% or lower.

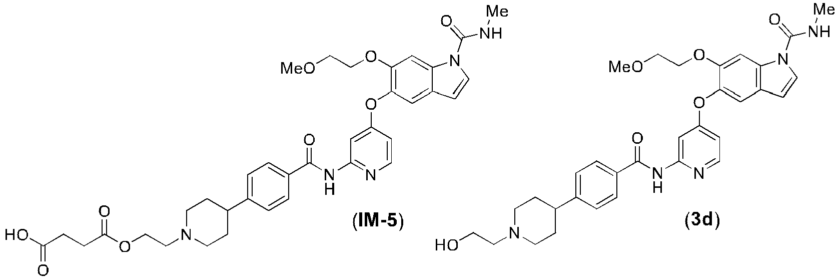

26. Compound (3d) or a salt thereof, wherein the content of compound (3d) or a salt thereof is 97.0 mass% or greater, the content of compound (IM-2) is 0.30 mass% or lower, and the content of all analogs is 2.0 mass% or lower.

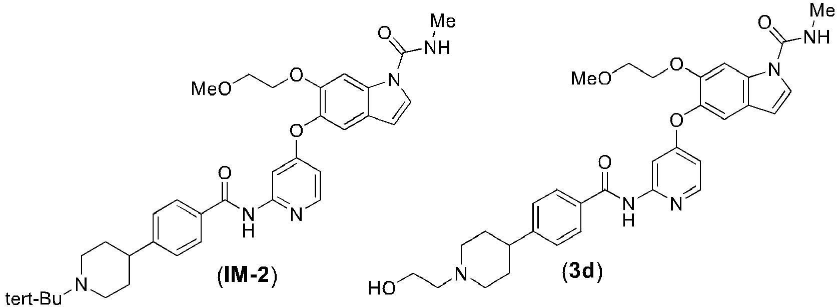

27. Compound (3d) or a salt thereof, wherein the content of compound (3d) or a salt thereof is 97.0 mass% or greater, the content of compound (3c) is 0.30 mass% or lower, and the content of all analogs is 2.0 mass% or lower.

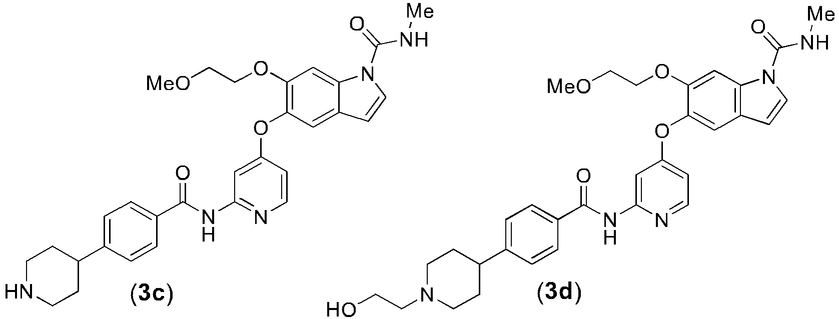

28. Compound (3d) or a salt thereof, wherein the content of compound (3d) or a salt thereof is 97.0 mass% or greater, the content of compound (IM-3) is 0.30 mass% or lower, and the content of all analogs is 2.0 mass% or lower.

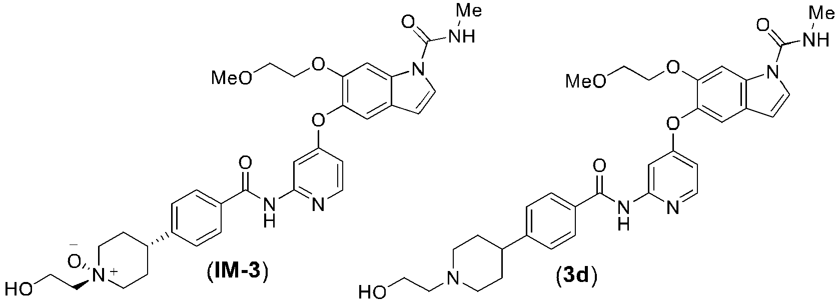

29. A 1.5 succinate of compound (3d), wherein the succinic acid content in the 1.5 succinate of compound (3d) is 20.8 mass% to 25.5 mass%.

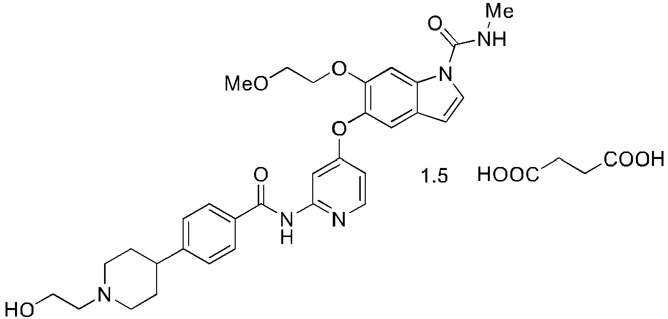

30. A 1.5 succinate of compound (3d), wherein the succinic acid content in the 1.5 succinate of compound (3d) is 21.9 mass% to 24.3 mass%.

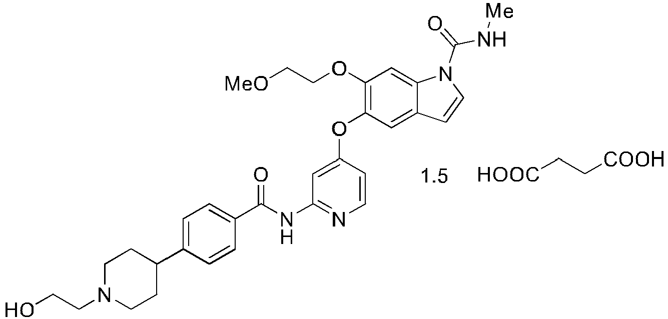

31. Compound (3d) or a salt thereof, wherein the content of compound (IM-2) is 0.15 mass% or lower.

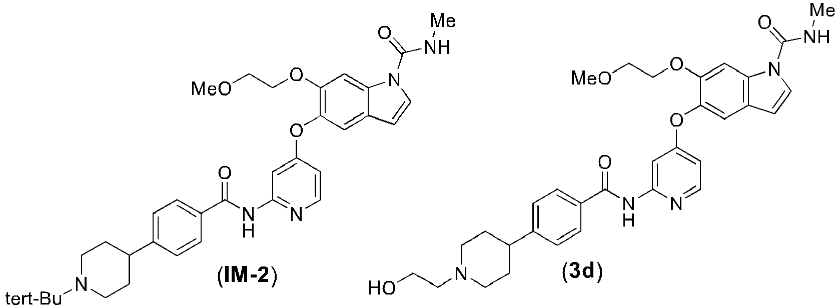

32. Compound (3d) or a salt thereof, wherein the content of compound (IM-3) is 0.15 mass% or lower.

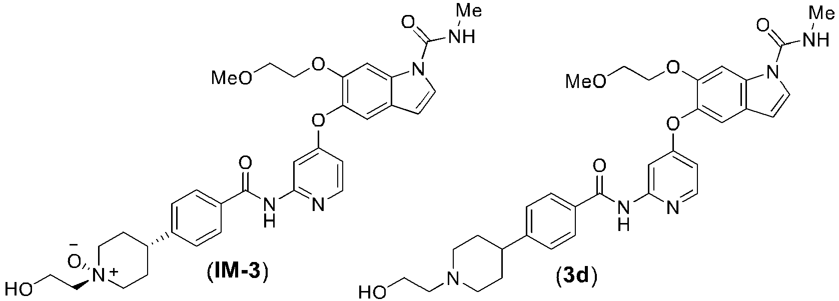

33. Compound (3d) or a salt thereof, wherein the content of compound (IM-2) is 0.15 mass% or lower and the content of all analogs is 2.0 mass% or lower.

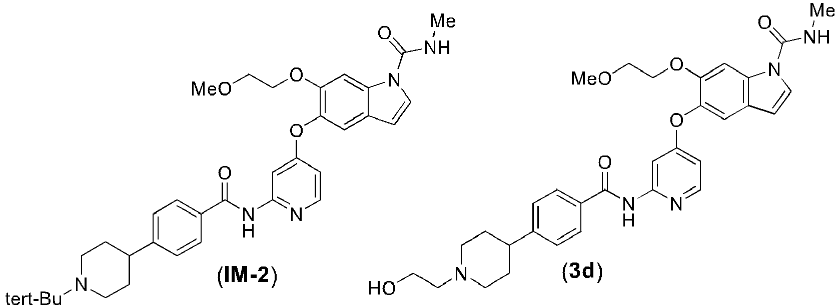

34. Compound (3d) or a salt thereof, wherein the content of compound (IM-3) is 0.15 mass% or lower and the content of all analogs is 2.0 mass% or lower.

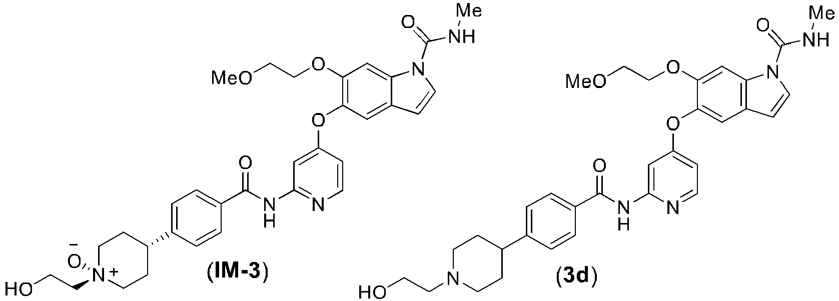

35. Compound (3d) or a salt thereof, wherein the content of compound (3d) or a salt thereof is 97.0 mass% or greater and the content of compound (IM-2) is 0.15 mass% or lower.

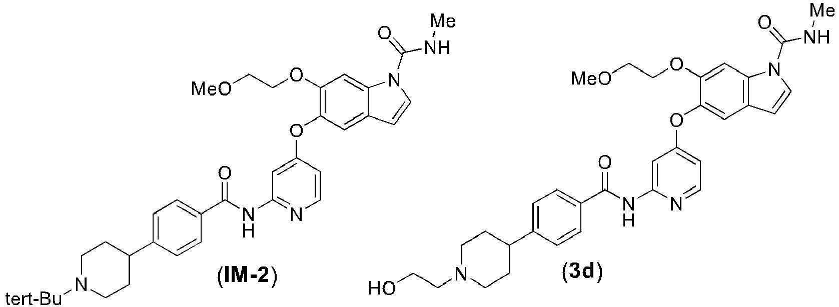

36. Compound (3d) or a salt thereof, wherein the content of compound (3d) or a salt thereof is 97.0 mass% or greater and the content of compound (IM-3) is 0.15 mass% or lower.

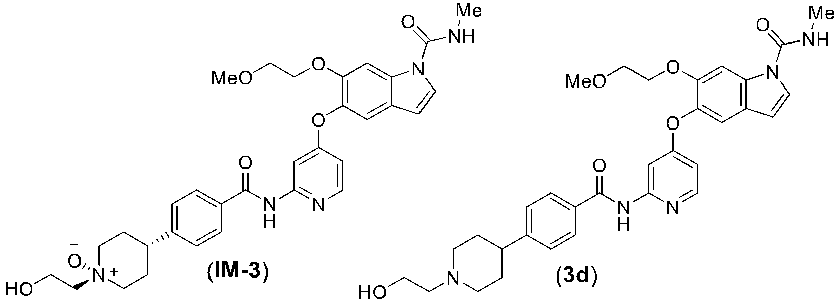

37. Compound (3d) or a salt thereof, wherein the content of compound (3d) or a salt thereof is 97.0 mass% or greater, the content of compound (IM-2) is 0.15 mass% or lower, and the content of all analogs is 2.0 mass% or lower.

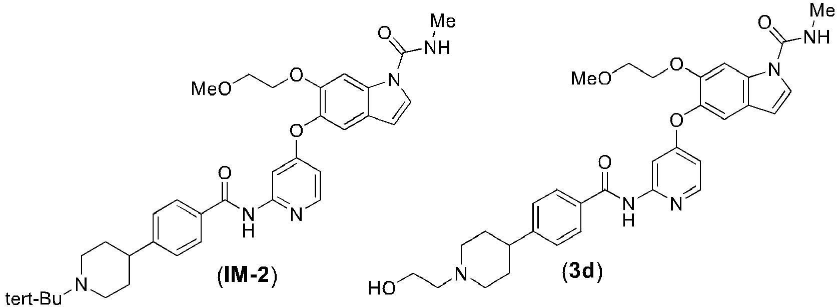

38. Compound (3d) or a salt thereof, wherein the content of compound (3d) or a salt thereof is 97.0 mass% or greater, the content of compound (IM-3) is 0.15 mass% or lower, and the content of all analogs is 2.0 mass% or lower.
